(19) Europäisches Patentamt
European Patent Office
Office européen des brevets

(11) Veröffentlichungsnummer: **0 315 182 B1**

(12) **EUROPÄISCHE PATENTSCHRIFT**

(45) Veröffentlichungstag der Patentschrift: **27.11.91**

(51) Int. Cl.5: **A61K 31/715**, A61K 35/78

(21) Anmeldenummer: **88118394.1**

(22) Anmeldetag: **04.11.88**

# (54) Arzneimittel mit einem Gehalt an einer Polysaccharide enthaltenden Komponente aus Thujapflanzen als aktivem Wirkstoff.

(30) Priorität: **06.11.87 DE 3738319**
**07.07.88 DE 3822945**

(43) Veröffentlichungstag der Anmeldung:
**10.05.89 Patentblatt 89/19**

(45) Bekanntmachung des Hinweises auf die Patenterteilung:
**27.11.91 Patentblatt 91/48**

(84) Benannte Vertragsstaaten:
**AT BE CH DE ES FR GB GR IT LI LU NL SE**

(56) Entgegenhaltungen:
**DE-A- 3 217 214**

**L. BEZANGER-BEAUQUESNE et al.: "Plantes médicinales des régions tempérées", 1980, Maloine S.A., Seiten 33,34, Paris, FR**

**ARZNEIMITTEL-FORSCHUNG, Band 35, Nr. 7, 1985, Seiten 1069-1075, Aulendorf, DE; H. WAGNER et al.: "Immunstimulierend wirkende Polysaccharide (Heteroglykane) aus höheren Pflanzen"**

(73) Patentinhaber: **Neth, Rolf Dietmar, Prof. Dr.**
**Pennskuhle 9**
**W-2110 Buchholz(DE)**

Patentinhaber: **STIFTUNG ZUR FÖRDERUNG DER ERFAHRUNGSHEILKUNDE IM STIFTERVERBAND FÜR DIE DEUTSCHE WISSENSCHAFT**
**Brucker Holt 56-60**
**W-4300 Essen 1(DE)**

(72) Erfinder: **Gohla, Sven**
**Waitzstrasse 17**
**W-2000 Hamburg 52(DE)**
Erfinder: **Neth, Rolf Dietmar, Prof. Dr.**
**Pennskuhle 9**
**W-2110 Buchholz(DE)**
Erfinder: **Haubeck, Hans-Dieter, Dr.**
**Veghestrasse 10**
**W-4400 Münster/Westf.(DE)**

(74) Vertreter: **UEXKÜLL & STOLBERG Patentanwälte**
**Beselerstrasse 4**
**W-2000 Hamburg 52(DE)**

EP 0 315 182 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft eine Polysaccharide enthaltende Komponente aus Thujapflanzen, insbesondere aus Thuja occidentale L., welche immun-modulierende Wirkungen insbesondere auf das spezifische Immunsystem von Säugetieren und Menschen besitzt.

Die Beeinflussung der lymphozytären Immunregulation durch Pflanzenextrakte war bisher nur als unspezifische polyklonale Lymphozytenstimulation durch beispielsweise die Stoffgruppe der sogenannten Lektine, d.h. mitogen wirkender Proteoglykane möglich. Nachteilig an den Lektinen ist ihre hohe Toxizität, die eine in vivo Applikation dieser Substanzen unmöglich macht.

Weiterhin wurden bisher Polysaccharidfraktionen, insbesondere aus Asteraceaen-Drogen beschrieben, die aus einem wäßrigen bzw. wäßrig-alkalischen Extrakt gewonnen wurden, (vgl. Wagner et al., Arzneimittelforschung, 35, 1069 bis 1075 (1985)). Diese Polysaccharidfraktionen zeigten eine unspezifische Steigerung der Granulozyten- und Monozyten- bzw. Makrophagen-Phagozytose. Eine schwache polyklonale Stimulation der B- und T-Lymphozytenfraktionen wurde ferner bei dem aus Echinacea purpurea isolierten Polysaccharid (EPS) beobachtet, vgl. Wagner et al., angewandte Phytotherapie 2, 166 (1981). Durch die beschriebenen Polysaccharide wird ferner eine polyklonale Stimulation der Mikro- und Makrophagozyten erreicht.

Es ist bisher kein Polysaccharid aus höheren Pflanzen beschrieben worden, welches eine selektive Stimulation der lymphoiden und/oder myoloiden Zellfraktionen bewirkt.

Ebenfalls war bisher eine selektive Beeinflussung spezifischer lymphozytärer Subpopolationen, wie beispielsweise der T-Helferzellen, in einem dem heutigen Stand der Forschung entsprechenden Umfang mit einer Polysaccharide enthaltenden Komponente aus höheren Pflanzen nicht möglich.

Es ist daher Aufgabe der Erfindung, Arzneimittel zur Behandlung von Erkrankungen zun schaffen, die mit einer Störung des Immunsystems einhergehen, wie dies z.B. bei dem Di-George-Syndrom und bei Aids der Fall ist.

Darüber hinaus soll ein neues Mittel zur Rekonstitution des Knochenmarks nach beispielsweise Einwirkung von ionisierenden Strahlen oder Cytostatica-Therapie geschaffen werden.

Insbesondere soll ein therapeutisches Mittel für Patienten mit HIV-Infektionen geschaffen werden, welches bei selektiver Rekonstitution der T-Helferzellen gleichzeitig eine Reduktion des die Krankheit verursachenden Virus HTLV IIIb bewirkt.

Zur Lösung der Aufgabe wird das Arzneimittel nach Anspruch 1 vorgeschlagen. Die Ansprüche 2 bis 6 betreffen bevorzugte Ausführungsformen.

Erfindungsgemäß hat es sich überraschenderweise gezeigt, daß eine Polysaccharide enthaltende Komponente aus Thujapflanzen, insbesondere aus Thuja occidentale L., einen mitogenen Effekt auf T-Helferlymphozyten besitzt. Diese Wirkung ist bei Fraktionen der Komponente mit einem Molekulargewicht von mehr als 100 000 $D^a$ besonders ausgeprägt (vgl. Beispiel 4).

Der erfindungsgemäße Einfluß auf die Untergruppe der T-Helferzellen mit Rezeptoren für die monoklonalen Antikörper Okt®4 und Okt®17 (vgl. Ortho-mune®, Monoklonale Antikörper, "Zur Typisierung immunologischer aktiver Zellen") ist selektiv (zur allgemeinen Charakterisierung der weißen Blutkörperchen vergleiche ferner Reinherz, E.L., Schlossman, S.F., "The Differentiation and function of human T lymphocytes, Cell 19, 821 bis 827 (1980) und Reinherz, E.L., et al. Leukocyte typing II, Band 3, "Human myeloid and hemapoietic cells", Springer Verlag, (1986) ).

Anders als mit den im Stand der Technik beschriebenen Polysaccharidfraktionen aus anderen höheren Pflanzen als Thuja, insbesondere aus den Asteraceaen-Drogen Echinacea angustifolia und purpurea wird mit den erfindungsgemäß isolierten, Polysaccharide enthaltenden Komponenten aus Thuja eine spezifische Induktion der T-Helferzellen erreicht.

Die Mitogenität der erfindungsgemäßen Komponenten ist abhängig von der Anwesenheit autologer Monozyten bzw. Makrophagen. Sie läßt sich weiterhin durch Anti-Interleukin-1-und Anti-Interferon-$\gamma$-Antikörper hemmen. Der selektiv-mitogene Effekt der Thujakomponente (TPS) ist verbunden mit einer erhöhten $^3$H-Thymidineinbaurate, die nach 7-tägiger Inkubation ihr Proliferationsmaximum erreicht.

Die durch TPS induzierten T-Helferzellen exprimieren die Oberflächenmarker Okt®4,, den Oberflächenmarker für T-Helferzellen, Okt®17, ein Oberflächenmarker der aktivierten Helferzellen und Okt®26a, den Interleukin-2 Rezeptor (zur Charaktierisierung der Oberflächenmarker vergleiche a.a.O.). Ferner wurde erfindungsgemäß eine TPS induzierte gesteigerte Produktion des Lymphokins Interleukin-2 beobachtet.

Zusätzlich wurde überraschenderweise gefunden, daß die erfindungsgemäße Thujakomponente unter gleichzeitiger Induktion von T-Helferzellen eine Verminderung der Replikation und Infektiösität von Lenti- und Retroviren gegenüber gesunden weißen Blutzellen von Vertrebraten bewirkt.

Schließlich hat es sich überraschenderweise gezeigt, daß die erfindungsgemäße Thujakomponente in der Lage ist, die Leukozytenbildung zu stimulieren und eine protektive Wirkung gegenüber radioaktiver Strahlenbelastung auszuüben (vgl. Beispiel 10).

Die erfindungsgemäße Polysaccharide enthaltende Thujakomponente kann aus den oberirdischen Pflanzenteilen von Thuja, vorzugsweise von Thuja occidentale L. nach dem Verfahren gemäß Caldes et al., J. Gen. Appl. Microbiol. 27, 157 ff. (1981) hergestellt werden.

Hierzu werden die Pflanzenteile zunächst zerkleinert, wie beispielsweise gemahlen. Vorzugsweise wird anschließend eine Vorextraktion mit organischen Lösungsmitteln wie Petrolether, Dichlormethan und/oder Methanol durchgeführt, um das Material aufzuschließen und zu reinigen.

Der Rückstand wird getrocknet und anschließend dem eigentlichen Extraktionsverfahren unterworfen, indem man ihn bei 1° bis 10° C, vorzugsweise bei 2° bis 6° C, mit einer wäßrigalkalischen Lösung extrahiert. Vorzugsweise verwendet man für die Extraktion 0,1 bis 1,0 N, insbesondere 0,5 N wäßrige NaOH-Lösung. Der Extrakt wird filtriert und anschließend mit Alkohol, vorzugsweise mit Ethanol ausgefällt, wobei ein Endverhältnis von Ethanol:Wasser von etwa 3:1 bevorzugt ist.

Der Niederschlag kann beispielsweise durch Ultrazentrifugation abgetrennt und anschließend in Wasser gelöst werden.

In der nächsten Stufe werden die Proteine durch Ansäuern der Lösung auf bekannte Weise beispielsweise mit Trichloressigsäure ausgefällt. Nach Abtrennen des Rückstandes wird der Überstand erneut mit Ethanol versetzt, um die erfindungsgemäße Polysaccharide enthaltende Komponente zu gewinnen.

Nach dem beschriebenen Verfahren werden im wesentlichen die Hemicellulosen erhalten. Die gewonnene Substanz wird in Wasser aufgenommen, durch beispielsweise ein Dialyserohr mit einer Trenngrenze von etwa 100 Dalton dialysiert und anschließend getrocknet, vorzugsweise gefriergetrocknet. Das erhaltene Produkt (TPS) ist im wesentlichen frei von Proteinen und Nukleotiden.

Zur weiteren Auftrennung kann TPS in eine wäßrige Lösung überführt und durch Ultrafiltration in Molekulargewicht bezogene Fraktionen unterteilt werden.

Erfindungsgemäß hat es sich gezeigt, daß sowohl die Gesamtfraktion TPS mit einem Molekulargewicht von mindestens etwa 100 $D^a$ als auch die TPS-Teilfraktionen die erfindungsgemäße Wirksamkeit aufweisen, wobei der mitogene Effekt vorzugsweise bei Fraktionen mit einem Molekulargewicht mit mindestens $1x10^5 D^a$ beobachtet wurde (vgl. Beispiel 3).

Überraschenderweise hat es sich zudem gezeigt, daß die erfindungsgemäße, Polysaccharide enthaltende Komponente aus Thujapflanzen auch in hohen Dosierungen keinerlei in vitrotoxische Wirksamkeit aufweist.

In in-vitro Experimenten mit menschlichen peripheren Blutzellen zeigte es sich, daß die optimale Wirksamkeit mit Wirkstoffkonzentrationen von etwa 0,1 bis 1 mg/ml wäßriger Lösung erreicht wird (vgl. Beispiel 3), wobei die Fraktionen mit höherem Molekulargewicht eine höhere spezifische Wirksamkeit aufweisen.

Die erfindungsgemäße Komponente aus Thujapflanzen TPS kann als Wirkstoff in Arzneimitteln verwendet werden, wobei zur Erzielung des mitogenen Effekts vorzugsweise Fraktionen mit einem Molekulargewicht von mindestens $1x10^5 D^a$ eingesetzt werden.

Der erfindungsgemäße Wirkstoff aus Thujapflanzen kann zusammen mit üblichen Träger- und Hilfsstoffen in Arzneimitteln verarbeitet werden, soweit letztere für die jeweilige Applikationsform geeignet und pharmazeutisch unbedenklich sind. Vorzugsweise wird TPS in physiologischer Kochsalzlösung parenteral verabreicht.

Die Erfindung wird nachfolgend anhand von Beispielen erläutert.

## Beispiel 1

### Herstellung der erfindungsgemäßen Komponente

Aufarbeitung der Droge:

Die getrocknete und gemahlene Droge wurde einer erschöpfenden Soxhlettextraktion mit Petrolether, Dichlormethan und Methanol unterzogen. Zur weiteren Aufarbeitung wurde die so vorextrahierte Droge an der Luft getrocknet.

Gewinnung der Rohpolysaccharide aus einem wäßrig-alkalischen Extrakt:

Alle Arbeiten wurden, wenn nicht anders vermerkt, bei 4° C durchgeführt.

800 g getrocknete, vorbehandelte Droge (s.o.) wurde mit 10 l einer 0,5 N NaOH versetzt und über Nacht gerührt.

Der Rückstand wurde abzentrifugiert, mit 5 l 0,5 NaOH nachgewaschen und die vereinigten Filtrate langsam unter ständigem Rühren mit dem dreifachen Volumen an 96%-igem Ethanol (unvergällt) versetzt. Der über Nacht gebildete Niederschlag wurde nun vorsichtig dekantiert und schließlich mittels Ultrazentrifugation von dem verbleibenden Überstand abgetrennt (15 000/UpM, t=15 min).

Der so erhaltene Niederschlag wurde in 3,5 l Wasser dest. gelöst und im Eisbad langsam mit 3,5 l 15%-iger Trichloressigsäure (TCA) versetzt und eine Stunde im Eisbad belassen. Danach wurde der gallertige Rückstand abzentrifugiert und der verbleibende Überstand, ca. 6 l, in 3 l Fraktionen aufgeteilt und mit je 12 l 96%-igen Ethanol tropfenweise versetzt. Nach 24 Stunden wurde der entstandene Niederschlag beider Fraktionen abzentrifugiert, vereinigt und in möglichst wenig 2%-iger Na-acetat - Lösung aufgenommen. Die unlöslichen Bestandteile wurden abzentrifugiert (10 000 g, t=20 min).

Die klare Lösung wurde mit 8 l 96%-igen Ethanol versetzt und der Niederschlag 4 Tage lang bei 4° C belassen. Danach wurde abzentrifugiert, das entstandene Rohpolysaccharid 96 Stunden lang gegen Wasser dest. dialysiert (Dialyserohr, Trenngrenze 100 $D^a$ ca. 4° C) und anschließend lyophilisiert.

Ausbeute: 15,66 g Rohsubstanz = 1,9%

Reinigung:

10 g des Rohpolysaccharides wurde in 500 ml Wasser dest. gelöst. Der Lösung wurde 500 ml 96%-iger Ethanol tropfenweise und unter ständigem Rühren bei 4° C hinzugefügt.

Der Niederschlag wurde nach 4 Tagen abzentrifugiert (15 000 UpM, 10 min) und der Überstand mit der gleichen Menge an Ethanol in gleicher Weise versetzt und weiter behandelt.

Der Vorgang wurde mit dem vierfachen Volumen an Ethanol wiederholt.

Die Niederschläge wurden in wenig Wasser dest. aufgenommen, dialysiert und anschließend gefriergetrocknet.

Ansatz: 10 g Rohsubstanz aus Thuja occidentale

TPS-Ausbeute: 1:1 Fällung: 8,76 g entsprechend 87,60% TPS bezogen auf die Rohsubstanz

TPS-Ausbeute: 1:4 Fällung: 0,767 g entsprechend 7,67% TPS bezogen auf die Rohsubstanz

**Beispiel 2**

**Isolierung von Subfraktionen aus TPS**

Die Isolation der Subfraktionen erfolgte mit Ultrafiltration. TPS gemäß Beispiel 1 wurde in Wasser dest. bis zu einer Konzentration von 2 mg/ml gelöst. 100 ml der Lösung wurden in einer Ultrafiltrationszelle (Ultra Sart 50, Sartorius, Deutschland) unter einem Stickstoffdruck von 1,5 bar kontinuierlich filtriert.

Dabei wurden die im folgenden aufgelisteten Filtertypen verwendet. Alle verwendeten Filtertypen wurden von der Firma Sartorius bezogen.

| Filtertyp | Trenngenze |
|---|---|
| SM 113 28 | >1000000$D^a$ |
| SM 113 18 | >300000$D^a$-<1000000$D^a$ |
| SM 146 69 | >100000$D^a$-<300000$D^a$ |
| SM 149 49 | >20000$D^a$-<100000$D^a$ |

Die so gewonnenen Subfraktionen wurden 48 Stunden lang gegen Aqua bidest dialysiert und anschließend lyophilisiert.

Ausbeute:

| | | |
|---|---|---|
| TPS 1, Mol Gew. | >1000000$D^a$ | 5,45 g |
| TPS 2, Mol Gew. | >300000$D^a$-1000000$D^a$ | 1,42 g |
| TPS 3, Mol Gew. | >100000$D^a$-<300000$D^a$ | 0,74 g |
| TPS 4, Mol Gew. | >20000$D^a$-<100000$D^a$ | 0,53 g |
| TPS 5, Mol Gew. | <20000$D^a$ | 0,25 g |

Dialysen:

Die Dialysen wurden mittels Dialyseschlauch (Union Carbide:Dialysis membrane, 1-8x100FP) oder eines Dialyserohres (Fa. Asahi Medical AM-100H) durchgeführt. Zur Dialyse im Dialyserohr wurde das zu dialysierende Material mittels einer Schlauchpumpe kontinuierlich durch das Rohr gepumpt. Am Wasserein- und Ausgangsstutzen des Dialyserohres wurde mittels einer Wasserstrahlpumpe (p nicht > 1,5 bar) die Dialyseflüssigkeit (Aqua dest.) ebenfalls kontinuierlich durch das Rohr geleitet. Aufgrund der hohen Trennleistung dieser Dialyserohre konnten die Dialysen auf 10 Stunden begrenzt werden.

Alle Subfraktionen sowie das Gesamt-TPS wurden nach Standardverfahren auf das Vorhandensein von Nukleotiden und Proteinen untersucht. Es zeigte sich, daß weder Proteine noch Nukleotide in nachweisbaren Mengen vorhanden waren.

Zur Bestimmung saurer Polysaccharide wurde der Carbazol-Test (vgl. Nature 1948, 483) verwendet. Zu diesem Zweck wurden Eichreihen von 5 bis 40 µg an Galakturonsäure und Glucuronsäure-τ-Lacton hergestellt.

Die zu untersuchenden Proben wurden mit $H_2SO_4$ aufgeschlossen und die Extinktion bestimmt. Anhand der Eichreihen wurde ein Uronsäuregehalt von etwa 5% festgestellt.

## Beispiel 3

## Untersuchung der mitogenen Wirkung von TPS sowie TPS 1 bis 4

TPS sowie die Subfraktionen TPS 1 bis 4 wurden in "Dulbecco's modified Eagle's medium (DME)" + 10% humanem AB-Serum (Sammelserum) gelöst. Die Wirkstoffkonzentration betrug jeweils 10 mg/ml.

Es wurde eine Wirkstoff-Verdünnungsreihe von 5 mg/ml bis 100 ng/ml hergestellt. Periphere humane Blutlymphozyten (PBL) wurden durch einen Polysaccharosegradienten (Ficoll-Paque, Pharmacia) angereichert, 5 Mal mit auf 37 °C temperierter phosphat-gepufferter Kochsalzlösung (0,9%) gewaschen, in DME + 10% AB-Serum aufgenommen und in einer Zelldichte von 5 x $10^5$ Zellen in 100 µl/Mikrotiterloch in eine 96-Loch-Flachbodenmikrotiterplatte (Fa. Greiner) verteilt.

Die Testlösungen wurden in den oben angegebenen Endkonzentrationen in Triplikaten ausgewertet. Die Inkubation erfolgte in einem Heraeus-Brutschrank bei 37 °C, 5% $CO_2$ und 100% Luftfeuchtigkeit. Als Blindwert fungierte 100 µl DME + 10% AB-Serum und als Positivwert wurde eine auf eine Endkonzentration von 10 µg/ml eingestellte Lektinlösung von Phaseolus spp., bekannt als Phythaemagglutinin, (PHA) verwendet.

Dieser Testansatz wurde bei allen Mitogennachweisen verwendet, wobei bei der Bestimmung der TPS-induzierten DNS-Synthese für TPS und die beschriebenen Kontrollkulturen folgen-der Inkubationsschritt zusätzlich ausgeführt wurde:
Pro Miktrotiterloch wurden in einer Endkonzentration/Loch von 0,2 bis 1,5 µCi $^3$H-Thymidin hinzugefügt und 12 Stunden lang im Inkubator bebrütet. Danach wurden die radioaktiv markierten Zellen mit einem Zellerntegerät (Scatron Cell-harvester) auf ein Filterpapier gesaugt und durch Zugabe von Szintillationscocktail (POPPOP) die Radioaktivität als cpm (counts per minute) bestimmt.

Die Ergebnisse sind in den Figuren 1 bis 4 wiedergegeben. Sie zeigen den mitogenen Effekt von TPS und TPS 1 bis 4 auf PBL.

## Beispiel 4

Die selektive Wirkung von TPS auf T-Helferzellen wurde anhand des APAAP-Verfahrens durch Färbung nachgewiesen (alkalische Phosphatase anti-alkalische Phosphatase-Färbung, Erber et al., Lanceti, 1042 ff.). Zunächst wurde anhand der Pappenheimfärbung (May Grünwald Giemsa Färbung) nachgewiesen, daß es sich bei den durch TPS stimulierten Zellen tatsächlich um Lymphoblasten handelte (vgl. Abb. 1). Die APAAP-Färbung ermöglichte im zweiten Schritt eine selektive Unterscheidung lymphozytärer sowie monozytärer Subpopulationen und somit eine Kategorisierung der Agenzwirkung auf spezifische Zellfraktionen (vgl. Abb. 2).

Als Gegenfärbemethode der APAAP-Färbung wurde die Hämatoxylin-Kernfärbung verwendet.

Für die Färbungen wurden die Zellen, die unter den Bedingungen gemäß Beispiel 3 kultiviert worden waren, mittels einer Eppendorfpipette aus den Mikrotiterlöchern geerntet und per Cytospin auf einem Objektträger fixiert. Ein Teil dieser Zellen wurde der Pappenheimfärbung unterzogen um festzustellen, welcher Grundtypus (monozytäre oder lymphozytäre Reihe) von Zellen durch TPS induziert wurden. Der andere Teil wurde mittels der APAAP-Methode auf spezifische lymphozytäre Zellmarker hin untersucht. Im einzelnen wurden folgende Antikörper verwendet:

Für T-Zellen: anti $Okt_3$ (anti-$CD_3$); anti $Okt_{11}$ (anti-$CD_2$);
anti $Okt_4$ (anti-$CD_4$) und anti $Okt_8$ (anti-$DC_8$).

Für B-Zellen: $OKB_{22}$ (anti-$CD_{22}$)

Die Ergebnisse sind in den Abbildungen 1 und 2 wiedergegeben. Sie zeigen, daß TPS die lymphoblastoiden Zellen zur Proliferation anregt (Abb. 1) und unter diesen die $CD_4$-positiven T-Zellen selektiv stimuliert (Abb. 2).

**Beispiel 5**

**Aktivierung der natürlichen Killerzellenantwort.**

**Herstellung einer Zellsuspension aus Milzen:**

Mäuse des Stammes BALB/C wurden durch cervikale Dislocation getötet. Die Milzen wurden steril entnommen und in einer Petrieschale (Greiner, Nürtingen), die mit 10 ml BSS (pH-stabilisierte Kochsalzlösung) gefüllt war, mit chirurgischen Pinzetten zerzupft.

Um eine Einzelzellsuspension zu erhalten, wurden die Zellen durch das Auf- und Abpipettieren mit einer Pasteurpipette aus den Gewebebruchstücken herausgespült. Die Suspension wurde durch ein steriles Drahtnetz filtriert, um Gewebereste zu beseitigen.

Die so präparierten Zellen wurden 5 x mit BSS gewaschen (200 g, 10 Min., Minifuge, Heraeus, Osterode).

Die Wirkung von TPS auf die Induktion von natürlichen Killerzellen wurde durch den $^{51}$Chromfreisetzungstest untersucht.

Herstellung der Zielzellen:

Die Milzzellen wurden wie beschrieben aufgearbeitet. YAC-1, ein Mausfibrosarkom, gilt als excellente NK-Zielzelle. Die Zellen wurden 3 x mit BSS gewaschen und auf etwa 5 x $10^6$ Zellen eingestellt. Diese Zellzahl wurde mit 0,1 ml $Na^{51}CrO_4$ (37 000 kilobequerel/ml, Amersham Buchler, Braunschweig) 1 Stunde lang bei 37° C inkubiert, anschließend 3 x mit BSS gewaschen und auf 4 x $10^5$ Zellen/Loch eingestellt. Diese Zellen wurden in 100 µl Schritten in 96-Loch-Flachboden Mikrotiterplatten ausplattiert.

Chromfreisetzung:

In einer Mikrotiterplatte wurden zu den Zielzellen unterschiedliche Mengen an Effektorzellen hinzugefügt, die zuvor über 6 bzw. 18 Stunden mit unterschiedlichen Konzentrationen an TPS inkubiert wurden. (100:1; 50:1; 25:1; 12,5:1.; 1 mg/ml-100ng/ml).

Das Endvolumen der Kultur betrug 0,2 ml. Die maximale $^{51}$Cr-Freisetzung wurde durch eine mit Natronlauge (1M, Merck) vermittelte totale Zellyse bestimmt. Die Zielzellen allein ergaben den Wert für die Spontanfreisetzung. Die Zellen wurden 6 bzw. 18 Stunden im Brutschrank bei 37° C inkubiert.

Messung:

Je 0,1 ml wurde aus jedem Ansatz entnommen und die darin enthaltene Radioaktivität im Gammazähler gemessen.

Die lytische Aktivität der Effektorzellen wurde als % Chromfreisetzung nach folgender Formel berechnet:

$$\% \text{ spezifische Lyse} = \frac{cpm_{Testansatz} - cpm_{Spontanfreisetzung}}{cpm_{max.\ Freisetzung} - cpm_{Spontanfreisetzung}} \times 100$$

Die Ergebnisse sind in Figur 5 wiedergegeben. Sie zeigen die Steigerung der Killerzellaktivität unter dem Einfluß von TPS gegenüber YAC-1-Zielzellen.

**Beispiel 6**

Die Granulozyten-Phagocytose-Steigerung durch TPS wurde nach Allen (vgl. Allen L.C. Et al, Biochem. Biophys. comp. Res. Immun.47, 679) bestimmt.

Die Ergebnisse sind in Figur 6 wiedergegeben. Sie zeigen die Phagocytosesteigerung neutrophiler Granulozyten für Zymosan unter dem Einfluß von TPS, wobei das Wirkungsoptimum bei 100 µg/ml liegt.

Als zweite Referenzmethode wurde der Granulozyten-Phagozytose-Assay nach Brandt, Scand. J. Haematol. Suppl., 2 (1967) zur panoptischen Bewertung der Granulozyten-Phygozytose herangezogen. Als Zielsubstanz wurden lebende ganze Zellen von Candida albicaus eingesetzt. Beide Methoden zeigten übereinstimmend die Phagotytosesteigerung neutrophiler Granulozyten unter dem Einfluß von TPS sowie der Teilfraktionen TPS1 und TPS2, wobei das Wirkungsoptimum bei jeweils 100 µg Polysaccharidendkonzentration/Reaktionsgefäß lag.

Die Ergebnisse dieser Versuche sind in den Figuren 7 und 8 wiedergegeben.

**Beispiel 7**

Die antivirale Wirksamkeit von TPS gegenüber Retroviren wurde im humanen in vitro Modell durch zwei unabhängige Testsysteme nachgewiesen.

Im 1. Testsystem wurde die Aktivität der reversen Transkriptase nach der Methode von B. Broiesz et al., Proc. Natl. Acad. Sci., USA 77, 7415 (1980), bestimmt. Die Aktivität der viruseigenen reversen Transscriptase dient als Hinweis auf die Virusaktivität. Sie wird anhand des Einbaus von 3H-Methylmarkiertem Dexoxythymidin-5′-triphosphat (TTP), gemessen.

Im 2. Testsystem wurde das den humanen Retroviren eigene Hüllprotein p24 durch einen "antigen-capture"-ELISA nach Popovic et al., Science 224, 497 (1984) bestimmt. Bei diesem Test ist die Farbintensität gemessen als Extinktion bei 410 nm proportional zur Konzentration des vorhandenen p24.

Für beide Testsysteme wurden periphere, mit HIV-Viren infizierte Blutleukozyten 3 Tage lang mit TPS vorstimuliert und anschließend 11 Tage lang mit Interleukin-2 Medium nachinkubiert. Nach insgesamt 14 Tagen wurde der Virustiter der Kulturüberstände dieser peripheren Blutleukozyten (PBL) sowie geeigneter zum Vergleich herangezogener Proben mittels beider Testverfahren bestimmt. Die Ergebnisse sind in den Tabellen 1 und 2 wiedergegeben. Sie zeigen, daß TPS unter Beibehaltung der mitogenen Eigenschaften auf T-Helferzellen und unreife T-Zellen bzw. Nullzellen einen hemmenden Effekt auf die Neuinfektion der durch TPS-Wirkung entstandenen T-Helferzellen und gleichzeitig eine deutliche Verminderung der eingesetzten Ausgangskonzentration des Virus bewirkt.

Tabelle 1

| Reverse Transkriptase Test nach Broiesz et al. | | |
|---|---|---|
| Versuchansatz | Aktivität des dTdA (DNA-Synthese) als cpm | Aktivität des dTrA (RNA-Synthese) als cpm |
| PBL mit 50000 Einheiten $HTLV_{IIIb}$ infiziert, ohne Stimulations des Wachstums | 2566 | 1789 |
| PBL nicht infiziert, mit PHA-P aktiviert | 2278 | 1868 |
| PBL mit 50000 Einheiten $HTLV_{IIIb}$ infiziert, mit PHA-P aktiviert | 3348 | 100445 |
| PBL mit 50000 Einheiten $HTLV_{IIIb}$ infiziert, mit TPS aktiviert | 2238 | 2194 |

Tabelle 2

| Antigen-capture Test nach Popovic et al. | |
|---|---|
| Versuchsansatz | Extinktion 410 nm |
| PBL nicht infiziert, ohne Stimulation des Wachstums | 0,046 |
| PBL nicht infiziert, mit PHA-P aktiviert | 0,035 |
| PBL nicht infiziert, mit TPS aktiviert | 0,044 |
| PBL mit 25000 Einheiten $HTLV_{IIIb}$ infiziert, ohne Stimulation des Wachstums | 0,061 |
| PBL mit PHA-P stimuliert, mit 25000 Einheiten $HTLV_{IIIb}$ infiziert | 0,353 |
| PBL mit TPS stimuliert, mit 25000 Einheiten $HTLV_{IIIb}$ infiziert | 0,051 |
| PBL ohne Stimulation des Wachstums, mit 50000 Einheiten $HTLV_{IIIb}$ infiziert | 0,059 |
| PBL mit PHA-P stimuliert, mit 50000 Einheiten $HTLV_{IIIb}$ infiziert | 0,804 |
| PBL mit TPS stimuliert, mit 50000 Einheiten $HTLV_{IIIb}$ infiziert | 0,055 |
| PBL ohne Stimulation des Wachstums, mit 300000 Einheiten $HTLV_{IIIb}$ infiziert | 0,256 |
| PBL mit PHA-P stimuliert, mit 300000 Einheiten $HTLV_{IIIb}$ infiziert | 2,819 |
| PBL mit TPS stimuliert, mit 300000 Einheiten $HTLV_{IIIb}$ infiziert | 0,187 |

**Beispiel 8**

Wirkung von TPS auf angereicherte B- und T-Lymphozytenfraktionen

Anreicherung von T-Zellen

Um T-Zellen anzureichern wurden periphere humane Blutleukozyten über einen Polysaccharosegradienten gewonnen und die monozytären Zellen nach dem Adherenzverfahren von Gartner et al. Science 233, 215 ff. (1986) entfernt.

Zur Anreicherung von T-Zellen wurde adaptiv das für Mäusemilzzellen von Julius et al., Eur. J. Immunol. 3, 6785 ff. (1973) beschriebene Auftrennungsverfahren angewendet.

Anreicherung von T-Zellfraktionen:

Nylonwolle (Leuko-Pak, Fenwall Lb., Derfield, USA) wurde vor dem Gebrauch 10x mit Aqua bidest. aufgekocht, um störende wasserlösliche Begleitstoffe quantitativ zu entfernen.

Die Wolle wurde anschließend getrocknet und staubfrei gelagert. In eine 10 ml Glasspritze wurden 1g der vorgereinigten Wolle gegeben, mit 5 ml Aqua bidest. versehen und anschließend autoklaviert, um eine Trennsäule herzustellen.

Die gebrauchsfertige Säule wurde nun mit einem sterilen Hahn und Kanüle bestückt. Im Anschluß daran wurde die Säule auf 37°C erwärmt, mit 75 ml BSS + 5% AB-Serum gespült und bei 37°C bei geschlossenem Hahn 1 Stunde lang inkubiert. Die Zellsuspension aus peripheren humanen Blutleukozyten wurde, wie zuvor angegeben, aufbereitet. Die nicht-adhärenten Zellen wurden aufgewirbelt und abzentrifugiert. Die so gewonnenen, nicht-adhärenten Zellen wurden in 2-3 ml BSS + 5% humanem AB-Serum aufgenommen und auf die Säule aufgebracht. Die beladene Säule wurde 1 Stunde lang bei 37°C im Brutschrank belassen. Die nicht an der Nylonwolle haftenden Zellen wurden vorsichtig abgespült, wobei ca. 35 ml Eluat aufgefangen wurden. Die so erhaltenen Zellen wurden für Testansätze verwendet, in denen angereicherte T-Zellfraktionen benötigt wurden.

Anreicherung von B-Zellfraktionen:

B-Zellfraktionen wurden erhalten, indem die humanen peripheren Blutleukozyten dem Polysaccharosegradienten der Adherenztrennung nach Gartner et al. (a.a.O.) sowie der oben beschriebenen Behandlung unterzogen wurden.

Die nicht eluierten, an der Nylon-Säule haftenden Zellen wurden mit 25 ml eiskalter PBS-Lösung ohne $Ca^{2+}$ und $Mg^{2+}$ abgespült.

Diese Zellen wurden in den Testsystemen als angereicherte B-Zellfraktionen verwendet.

Die Wirkung von TPS auf die DNA-Synthese-Rate wird anhand des Einbaus von 3H-Thymidin gemessen. Als Testmedium wird RPMI-1640-Medium supplementiert mit 10%-igem humanem AB-Serum verwendet.

Angereicherte T- und B-Zellfraktionen sowie PBL wurden in einer Konzentration von $5x10^5$ Zellen pro Loch einer 96-Loch Flachbodenmikrotiterplatte ausgesät. Die autologen Monozyten wurden in einer Konzentration von $5x10^4$ Zellen den entsprechenden Proben hinzugefügt. TPS wurde in einer Endkonzentration von 1 mg/ml je Loch eingesetzt. Die Platten wurden 4 Tage lang bei 37°C inkubiert, wobei während der letzten 12 Stunden 0,5 Ci/Loch Mikrotiterplatte vorhanden waren.

Die Einbaurate wurde anschließend nach dem in Beispiel 3 beschriebenen Verfahren bestimmt.

Die Ergebnisse sind in den Figuren 9 und 10 wiedergegeben. Sie zeigen, daß TPS keine nennenswerte Wirkung auf angereicherte B-Lymphozyten-Fraktionen ausübt, während die T-Lymphozyten in Gegenwart von Monozyten bzw. Makrophagen selektiv stimuliert werden. Dieses Ergebnis bestätigt die in Beispiel 4 beschriebenen Ergebnisse.

**Beispiel 9**

Untersuchung der Interleukin-2 Produktion und der Expression des Interleukin-2-Rezeptors als Parameter der T-Helferzell-Aktivierung.

Der anti-Interleukin-2 Test wurde gemäß der Herstellerinformation der Firma Genzyme Corp., USA, als Interest $2^R$ durchgeführt. Als Testfraktion wurden Zellkulturüberstände (Konditionierte Medien) peripherer Blutleukozytenfraktionen verwendet, welche zuvor 4 Tage lang in DME + 10% AB-Serum inkubiert worden waren.

Durchführung der Untersuchung:

Zwei Tage vor Testbeginn wurde die Mikrotiterplatte mit monoklonalem anti-Interleukin-2 Antikörpern beschichtet. Hierzu wurden die monoklonalen Antikörper in 11,5 ml "coating buffer" resuspendiert. 100 µl dieser Lösung wurden pro Loch einer Mikrotiterplatte (Genzyme) verteilt. Anschließend wurde die Platte in einer feuchten Kammer bei 4° C über den oben angegebenen Zeitraum inkubiert.

Am Tag des Testes wurde der Interleukin-2 Standard, bestehend aus rekombinantem humanem Il-2 (rIL-2), mit DME + 10% AB Serum rekonstituiert und die folgende Verdünnungsreihe hergestellt: Das lyophilisierte rIl-2 wurde in 1 ml des Kulturmediums aufgenommen. Diese Lösung entsprach einer Il-2 Konzentration von 500 U/ml. 0,1 ml wurden dieser Lösung entnommen und auf 1 ml mit "coating buffer" aufgefüllt.

Dieser Vorgang wurde dreimal nacheinander so wiederholt, daß 0,1 ml der vorhergehenden Standardverdünnung entnommen und mit "coating buffer" auf 1 ml aufgefüllt wurden. Man erhielt daraufhin eine Konzentrationsreihe von 500 U/ml bis 0,5 U/ml rIl-2.

Nach dem Beschichten mit rIl-2 wurde dreimal mit Waschpuffer gewaschen. Anschließend wurden die Interleukin-2 Standardverdünnungen in Duplikaten a 100 µl/Loch einer Mikrotiterplatte verteilt. Als Negativkontrolle fungierten je 100 µl/Loch Kulturmedium, das unter den gleichen Bedingungen inkubiert wurde, die auch für die Herstellung der konditionierten Medien galten. Die Platte wurden anschließend bei 37° C für eine Stunde lang inkubiert. Danach wurde erneut dreimal mit Waschpuffer gewaschen.

Währenddessen wurde der zweite Antikörper, ein polyvalenter Kaninchen anti-human IL-2 Antikörper, vorbereitet, indem 0,3 ml der Antikörperlösung mit 14 ml PBS/Tween-Waschpuffer versetzt wurden. Diese Lösung wurde in 100 µl Schritten pro Mikrotiterloch verteilt. Die Inkubation erfolgte 1 Stunde lang bei 37° C.

Anschließend wurde wieder dreimal mit PBS/Tween-Waschpuffer gewaschen und mit dem dritten Antikörper inkubiert. Bei diesem handelt es sich um einen mit alkalischer Phosphatase konjugierten Ziegen anti-Kaninchen Antikörper. 200 µl dieses Antikörperkonjugates wurden mit 12 ml PBS/Tween Waschpuffer gemischt und je 100 µl/Loch verteilt.

Die Inkubation erfolgte eine Stunde lang bei 37° C.

Nach dreimaligem Waschen mit PBS/Tween-Waschpuffer wurde mit dem Substrat, bestehend aus

| | |
|---|---|
| Substratpuffer | 6 ml |
| $MgCl_2$-Lösung | 6 ml |
| p-NPP-Substrat | 10mg, |

welches a 100 µl/Loch verteilt wurde, eine Stunde lang bei 20° c inkubiert.

Die enzymatische Freisetzung von p-Nitrophenol aus p-NPP ist proportional der Menge an gebundenem Interleukin-2.

Die Quantifizierung der Meßergebnisse erfolgte in einem ELISA-Reader (Dynatech) bei einer Wellenlänge von 410 nm.

Die Ergebnisse sind in den Tabellen 3 und 4 als Mittelwerte der jeweiligen Messungen wiedergegeben. Sie zeigen, die TPS-induzierte Steigerung der Interleukin-2 Produktion.

Tabelle 3

| Standardverdünnung für IL-2 | Extinktion |
|---|---|
| Kurvenstandards | Kontrollen |
| Interleukin-2 500U | 0,667 |
| Interleukin-2 50U | 0,350 |
| Interleukin-2 5U | 0,203 |
| Interleukin-2 0,5U | 0,154 |
| Interleukin-2 0,05U | 0,063 |
| Interleukin-2 0 U | 0,003 |

Tabelle 4

| Mitogen-Ansatz | Extinktion | c(U/ml) |
|---|---|---|
| PHA-P (10 µg/ml) | 0,229 | 16,5 |
| TPS (1 mg/ml) | 0,276 | 24,0 |
| Kontrolle (RPMI 1640 + 10% AB-Serum) | 0,158 | 0,83 |

**Beispiel 10**

Bestimmung des Wirkungsmechanismus von TPS:

Interleukin-1 und Interferon-τ sind wesentliche Zytokine, die eine entscheidende Rolle bei der Aktivierung von T-Zellen und Monozyten spielen. Während Interferon-τ nur von aktivierten T-Zellen produziert wird, erfolgt die Produktion von Interleukin-1 vor allem in Monozyten bzw. Makrophagen. Durch Antikörper gegen Interferon-τ und Interleukin-1 läßt sich die T-Zell-Aktivierung spezifisch dosisabhängig nahezu vollständig blockieren. Im Gegensatz hierzu wird durch Kontrollantikörper selbst in sehr hohen Konzentrationen eine vergleichsweise nur sehr geringe unspezifische Inhibition erreicht.

Durchführung:

Periphere humane Blutleukozyten wurden über einen Polysaccharosegradienten gewonnen und in einer Konzentration von $5 \times 10^5$ Zellen pro Loch einer Mikrotiterplatte ausgesät. TPS wurde in DME-Medium, supplementiert mit 10% humanem AB-Serum gelöst und in einer Endkonzentration von 1 mg/ml zum Test eingesetzt. Die Aktivität von TPS wurde anhand der 3H-Thymidineinbaurate wie in dem Beispiel 3 beschrieben gemessen.

Die spezifischen anti-Interleukin-1 und γ-Interferon-Antikörper wurden von der Firma Genzyme bezogen. Sie wurden vor Testbeginn 48 Stunden lang unter sterilen Bedingungen gegen Wasser dest. dialysiert, um das Konservierungsmittel Natriumazid quantitativ zu entfernen. Anschließend wurde der Titer der Antikörper auf 200 U/100 µl eingestellt, und es wurden die Antikörper in einer titrierten Endkonzentration von 100 U/bis 1U/Loch einer 96-Loch Flachbodenmikrotiterplatte in einem Volumen von 100 µl DME-Medium + 10% AB-Serum vorgelegt. Periphere Blutleukozyten sowie TPS wurden in 50 µl supplementiertem DME-Medium hinzugefügt. Die Platten wurden anschließend unter Standardbedingungen in Brutschrank inkubiert.

Die Ergebnisse sind in Tabelle 5 und Figur 11 wiedergegeben.

Sie zeigen, daß die mitogene Wirkung von TPS auf PBL durch hohe Dosen an anti-IL-1 und anti-µ-IFN stark vermindert werden kann. Ferner konnte nachgewiesen werden, daß dieser Effekt nicht auf einer unspezifischen Rezepturblockade beruht.

Tabelle 5

| Testansatz | cpm |
|---|---|
| PBL + TPS (1mg/ml)<br>PBL ohne TPS<br>PBL + 100 U anti-IL-1 ohne TPS<br>PBL + 100 U anti IL-1 + TPS (1mg/ml)<br>PBL + 10 U anti-IL-1 + TPS (1mg/ml)<br>PBL + 1 U anti-IL-1 + TPS (1 mg/ml)<br>PBL + 100 U anti-Interferon-τ ohne TPS<br>PBL + 100 U anti-Interferon-τ + TPS (1mg/ml)<br>PBL + 10 U anti/Interferon-τ + TPS (1mg/ml)<br>PBL + 1 U anti-Interferon-τ + TPS (1mg/ml)<br>PBL + Maus-IgG + TPS (1mg/ml)<br>PBL + IgG-Fraktion human + TPS (1 mg/ml) | 145137 +/- 1523<br>16303 +/- 534<br>27069 +/- 749<br>33047 +/- 871<br>49985 +/- 1003<br>82913 +/- 1324<br>37738 +/- 656<br>43875 +/- 637<br>46726 +/- 527<br>78681 +/- 799<br>100807 +/- 1956<br>107765 +/- 2097 |

## Beispiel 11

Nachweis der in vivo immummodulativen Wirkung von TPS an BALB-C-Inzuchtmäusen

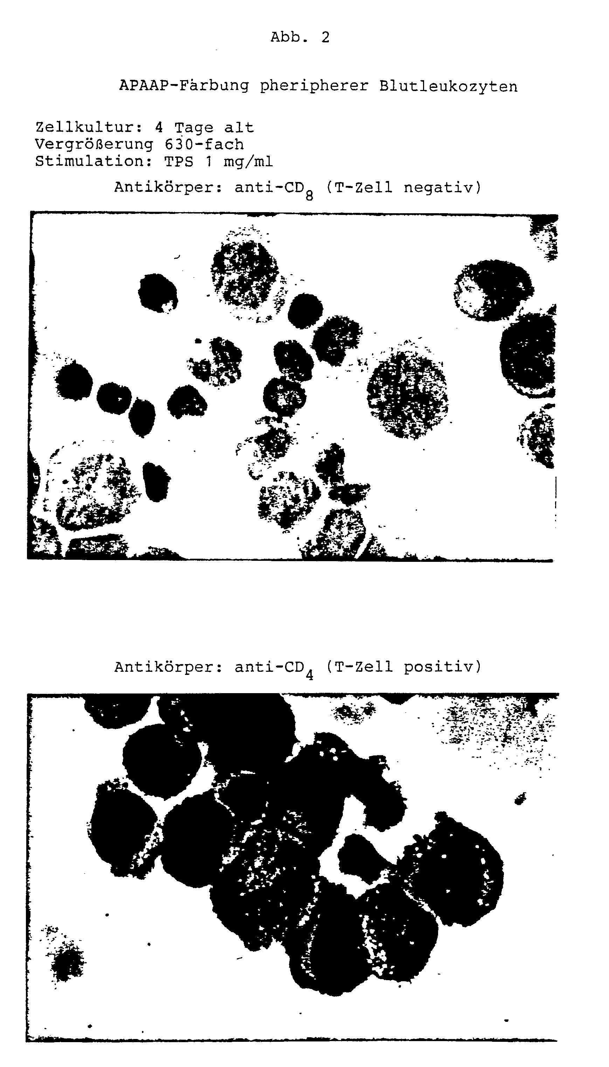

Der Versuch wurde in Anlehnung an Moore et al. (M.A.D. Moore, "Stolman Lecture", "Modern Trends of Human Leukemia", Wilsede (1988), Springer Verlag in press) durchgeführt. Je 10 BALB-C-Mäuse wurden zu einem Kollektiv zusammengefaßt und entweder intravenös mit 0,5 ml RPMI 1640 Medium oder mit TPS, gelöst in RPMI 1640 Medium, Endkonzentration 1 mg/500 µl/Tier, behandelt. Je 5 Tiere des TPS Kollektivs sowie des RPMI 1640 Kontrollkollektivs wurden einen Tag nach der Behandlung subletal mit 600 rad aus einer Kobaltbombe bestrahlt. Das Blut aller Tiere wurde vor der Behandlung zur Bestimmung von Normalwerten, sowie nach der Behandlung mit TPS oder mit Medium und nach der Bestrahlung durch Punktion des retroorbitalen Venenplexus gewonnen. Aus dem so gewonnenen Blut wurden anschließend mit Hilfe einer Neubauerkammer die Leukozytenzahl pro nl bestimmt, indem durch die Behandlung mit Gentiana-Violett die roten Blutkörperchen zerstört wurden und die Zahl der Leukozyten durch Auszählen der Kammer bestimmt wurde. Die Ergebnisse sind in Figur 12 wiedergegeben. Sie zeigen, daß auch in vivo eine TPS bedingte Steigerung der Leukozytenzahl im Vergleich zum RPMI 1640 Standard auftritt. Auch nach subletaler Bestrahlung weist das Kollektiv der TPS behandelten Mäuse eine höhere Leukozytenzahl auf als das RPMI behandelte Kontrollkollektiv.

## Patentansprüche

## Patentansprüche für folgende Vertragsstaaten : AT, BE, CH, DE, FR, GB, IT, LI, LU, NL, SE

1. Arzneimittel mit einem Gehalt an einer Polysaccharide enthaltenden Komponente aus Thujapflanzen, welche erhältlich ist durch
   (a) Extrahieren der zerkleinerten und mit organischen Lösungsmitteln gereinigten oberirdischen Thujapflanzenteile mit einem alkalisch-wäßrigen Lösungsmittel,
   (b) Versetzen des Extrakts mit einem organischen Lösungsmittel, um Polysaccharide und Proteine aus dem Extrakt auszufällen,
   (c) Abtrennen des Niederschlags und Lösen des Niederschlags in Wasser bei Temperaturen unterhalb Raumtemperatur,
   (d) Ansäuern der Lösung, um die Proteine auszufällen,
   (e) Abtrennen des Niederschlags und Versetzen des Überstandes mit einem organischen Lösungsmittel,
   (f) Dialysieren des Niederschlags und anschließendes Trocknen.

2. Arzneimittel nach Anspruch 1, **dadurch gekennzeichnet,** daß das Molekulargewicht der Komponente mindestens 100 D[a] beträgt.

3. Arzneimittel nach Anspruch 1, **dadurch gekennzeichnet,** daß das Molekulargewicht der Komponente gemäß Ultrafiltration mindestens 100 000 Da beträgt.

4. Zusammensetzung nach den Ansprüchen 1 bis 3, **dadurch gekennzeichnet,** daß die Polysaccharid enthaltende Komponente aus Thuja occidentale L. stammt.

5. Arzneimittel nach den Ansprüchen 1 bis 4 zur Behandlung von Immun- oder Knochenmarksdepressionen.

6. Arzneimittel nach Anspruch 5 zur Behandlung von endogenen und erworbenen, einschließlich Virusinduzierten Immundefekterkrankungen.

**Patentansprüche für folgende Vertragsstaaten : ES, GR**

1. Verfahren zum Herstellen von Arzneimitteln mit einem Gehalt an einer Polysaccharide enthaltenden Komponente aus Thujapflanzen, durch

   a) Extrahieren der zerkleinerten und mit organischen Lösungsmitteln gereinigten oberirdischen Thujapflanzenteile mit einem alkalisch-wäßrigen Lösungsmittel,
   b) Versetzen des Extrakts mit einem organischen Lösungsmittel, um Polysaccharide und Proteine aus dem Extrakt auszufällen,
   c) Abtrennen des Niederschlags und Lösen des Niederschlags in Wasser bei Temperaturen unterhalb Raumtemperatur,
   d) Ansäuern der Lösung, um die Proteine auszufällen,
   e) Abtrennen des Niederschlags und Versetzen des Überstandes mit einem organischen Lösungsmittel,
   f) Dialysieren des Niederschlags und anschließendes Trocknen.

2. Verfahren nach Anspruch 1, **dadurch gekennzeichnet,** daß das Molekulargewicht der Komponente mindestens 100Da beträgt.

3. Verfahren nach Anspruch 1, **dadurch gekennzeichnet,** daß das Molekulargewicht der Komponente gemäß Ultrafiltration mindestens 100 000 Da beträgt.

4. Verfahren nach den Ansprüchen 1 bis 3, **dadurch gekennzeichnet,** daß die Polysaccharid enthaltende Komponente aus Thuja occidentale L. stammt.

5. Verwendung der nach dem Verfahren gemäß den Ansprüchen 1 bis 4 hergestellten, Polysaccharide enthaltenden Komponente aus Thujapflanzen zur Herstellung von Arzneimitteln zur Behandlung von Immun- oder Knochenmarksdepressionen.

6. Verwendung der nach dem Verfahren gemäß den Ansprüchen 1 bis 4 hergestellten, Polysaccharide enthaltenden Komponente aus Thujapflanzen zur Herstellung von Arzneimitteln zur Behandlung von endogenen und erworbenen, einschließlich Virusinduzierten Immundefekterkrankungen.

**Claims**

**Claims for the following Contracting States : AT, BE, CH, DE, FR, GB, IT, LI, LU, NL, SE**

1. Medicament containing a component from thuja plants, which contains polysaccharides, which can be obtained by

   (a) extraction using an alkaline-aqueous solvent of the crushed, above-ground thuja plant parts, which are cleaned with organic solvents,
   (b) mixture of the extract with an organic solvent, in order to precipitate polysaccharides and proteins from the extract,
   (c) separation of the precipitate and dissolution of the precipitate in water at a temperature below ambient temperature,
   (d) acidification of the solution in order to precipitate the proteins,
   (e) separation of the precipitate and mixing of the supernatant with an organic solvent,
   (f) dialysis of the precipitate and subsequent drying.

2. Medicament according to claim 1, **characterized in that** the molecular weight of the component is at least 100 $D^a$.

**3.** Medicament according to claim 1, **characterized in that** the molecular weight of the component according to ultra-filtration is at least 100, 000 $D^a$.

**4.** Composition according to claims 1 to 3, **characterized in that** the component containing polysaccharide originates from Thuja occidentale L.

**5.** Medicament according to claims 1 to 4 for the treatment of immuno- or bone-marrow depressions.

**6.** Medicament according to claim 5 for the treatment of endogenous and acquired, including virus-induced, immune deficiency diseases.

**Claims for the following Contracting States : ES, GR**

**1.** Process for producing medicaments containing a component from thuja plants, which contains polysaccharides, by

(a) extraction using an alkaline-aqueous solvent of the crushed, above-ground thuja plant parts, which are cleaned with organic solvents,
(b) mixture of the extract with an organic solvent, in order to precipitate polysaccharides and proteins from the extract,
(c) separation of the precipitate and dissolution of the precipitate in water at a temperature below ambient temperature,
(d) acidification of the solution in order to precipitate the proteins,
(e) separation of the precipitate and mixing of the supernatant with an organic solvent,
(f) dialysis of the precipitate and subsequent drying.

**2.** Process according to claim 1, **characterized in that** the molecular weight of the component is at least 100 $D^a$.

**3.** Process according to claim 1, **characterized in that** the molecular weight of the component according to ultra-filtration is at least 100, 000 $D^a$.

**4.** Process according to claims 1 to 3, **characterized in that** the component containing polysaccharide originates from Thuja occidentale L.

**5.** Use of the component from thuja plants, which contains polysaccharides, produced using the process according to claims 1 to 4 to produce medicaments for the treatment of immuno- or bone-marrow depressions.

**6.** Use of the component from thuja plants, which contains polysaccharides, produced using the process according to claims 1 to 4 to produce medicaments for the treatment of endogenous and acquired, including virus-induced, immune deficiency diseases.

**Revendications**
**Revendications pour les Etats contractants suivants : AT, BE, CH, DE, FR, GB, IT, LI, LU, NL, SE**

**1.** Médicament contenant un composant polysaccharidique de plantes de thuya que l'on peut obtenir par
(a) extraction des parties de plantes de thuya au-dessus du sol qui sont mises en menus morceaux et nettoyées au moyen de solvants organiques, par un solvant alcalin aqueux ,
(b) mélange de l'extrait avec un solvant organique pour la précipitation des polysaccharides et des protéines, de l'extrait ,
(c) séparation du précité et dissolution du précipité dans l'eau à des températures en dessous de la température ambiante ,
(d) acidification de la solution, pour la précipitation des protéines ,
(e) séparation du précipité et mélange du résidu avec un solvant organique,
(f) dialyse du précipité et ensuite son séchage.

2. Médicament selon la revendication 1, caractérisé en ce que le poids moléculaire du composant est d'au moins 100 $D^a$.

3. Médicament selon la revendication 1, caractérisé en ce que le poids moléculaire du composant d'après l'ultrafiltration,est d'au moins 100.000 $D^a$.

4. Composition selon les revendications 1 à 3, caractérisée en ce que le composant polysaccharidique provient de Thuya occidentale L.

5. Médicament selon les revendications 1 à 4 pour le traitement des dépressions immunitaires ou de la moelle osseuse.

6. Médicament selon la revendication 5 pour le traitement de maladies de défaut immunitaire endogènes ou acquises, y compris induites par un virus.

**Revendications pour les Etats contractants suivants : ES, GR**

1. Procédé pour la production de médicaments contenant un composant polysaccharidique de plantes de thuya par
   (a) extraction des parties de plantes de thuya au-dessus du sol qui sont mises en menus morceaux et nettoyées au moyen de solvants organiques, par un solvant alcalin aqueux,
   (b) mélange de l'extrait avec un solvant organique pour la précipitation des polysaccharides et des protéines, de l'extrait,
   (c) séparation du précipité et dissolution du précipité dans l'eau à des températures en dessous de la température ambiante,
   (d) acidification de la solution, pour la précipitation des protéines,
   (e) séparation du précipité et mélange du résidu avec un solvant organique,
   (f) dialyse du précipité , et ensuite son séchage.

2. Procédé selon la revendication 1, caractérisé en ce que le poids moléculaire du composant est d'au moins 100 $D^a$.

3. Procédé selon la revendication 1, caractérisé en ce que le poids moléculaire du composant, d'après l'ultrafiltration,est d'au moins 100.000 $D^a$.

4. Procédé selon les revendications 1 à 3, caractérisé en ce que le composant polysaccharidique provient de Thuya occidentale L.

5. Utilisation du composant polysaccharidique obtenu par le procédé selon les revendications 1 à 4 à partir de plantes de thuya, pour la production de médicaments pour le traitement de dépressions immunitaires ou de la moelle osseuse.

6. Utilisation du composant polysaccharidique obtenu par le procédé selon les revendications 1 à 4, provenant des plantes de thuya, pour la production de médicaments pour le traitement de maladies immunodéficitaires endogènes ou acquises, y compris induites par un virus.

FIGUR 1

Mitogen-Nachweis (Kinetik)

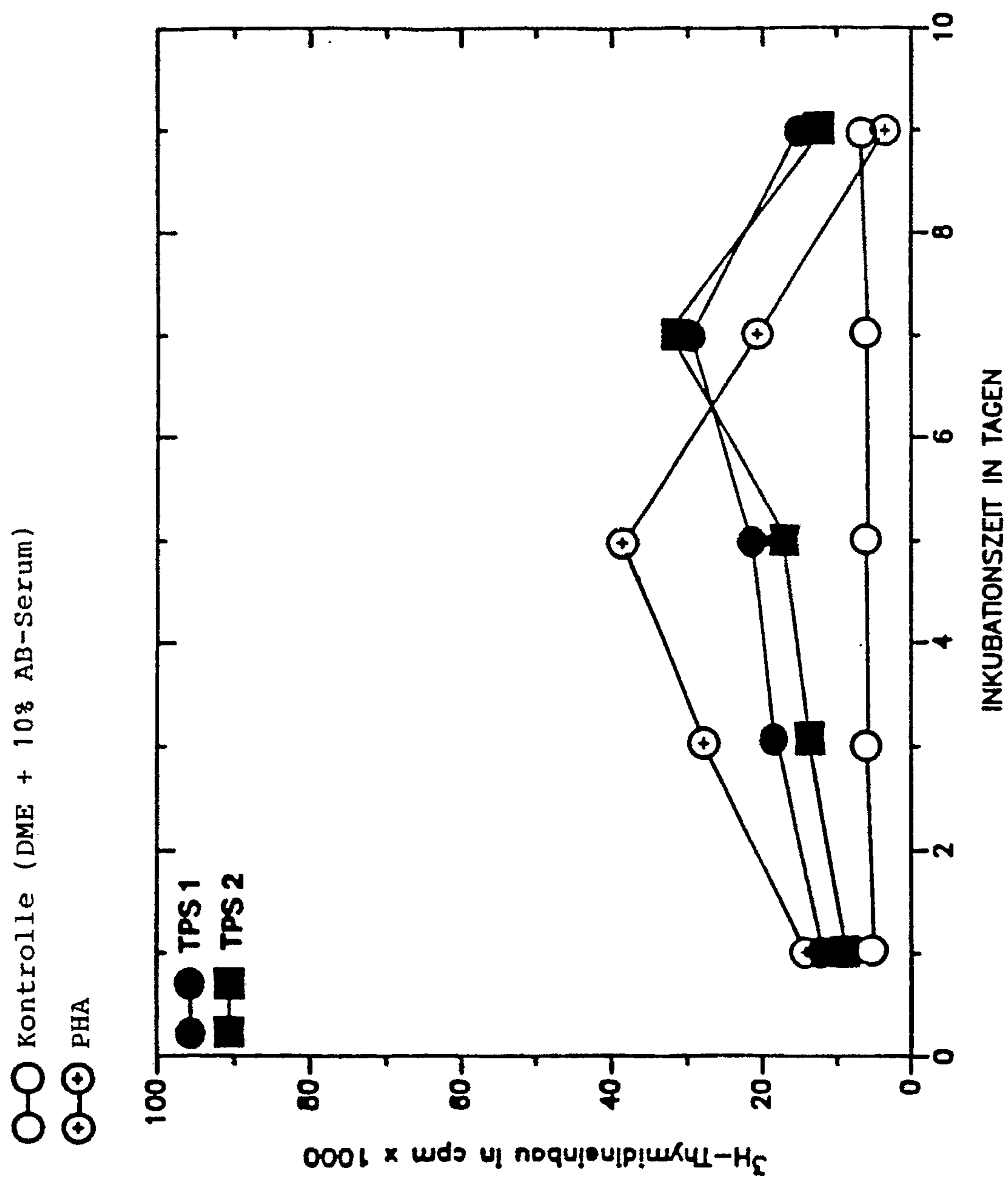

FIGUR 2

Zeit und Dosierungsabhängigkeit der mitogenen Wirkung von TPS

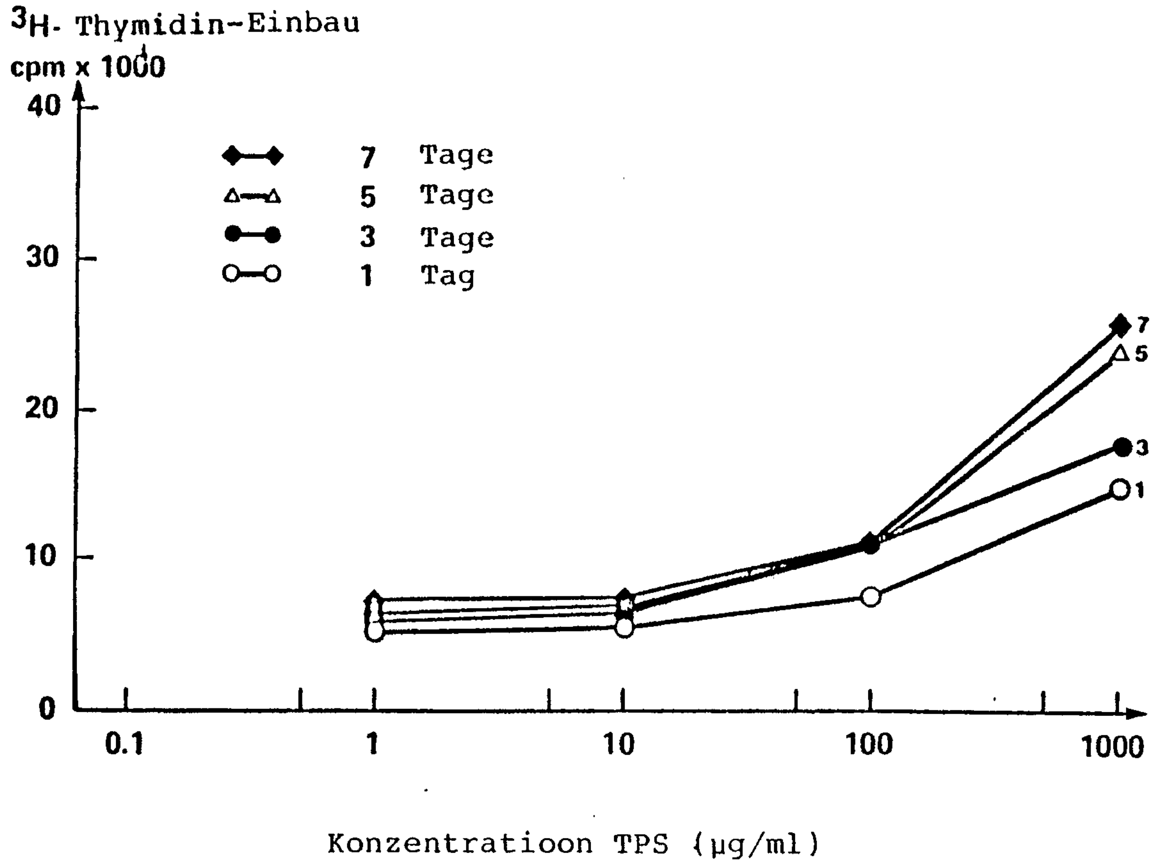

FIGUR 3

Mitogen-Nachweis

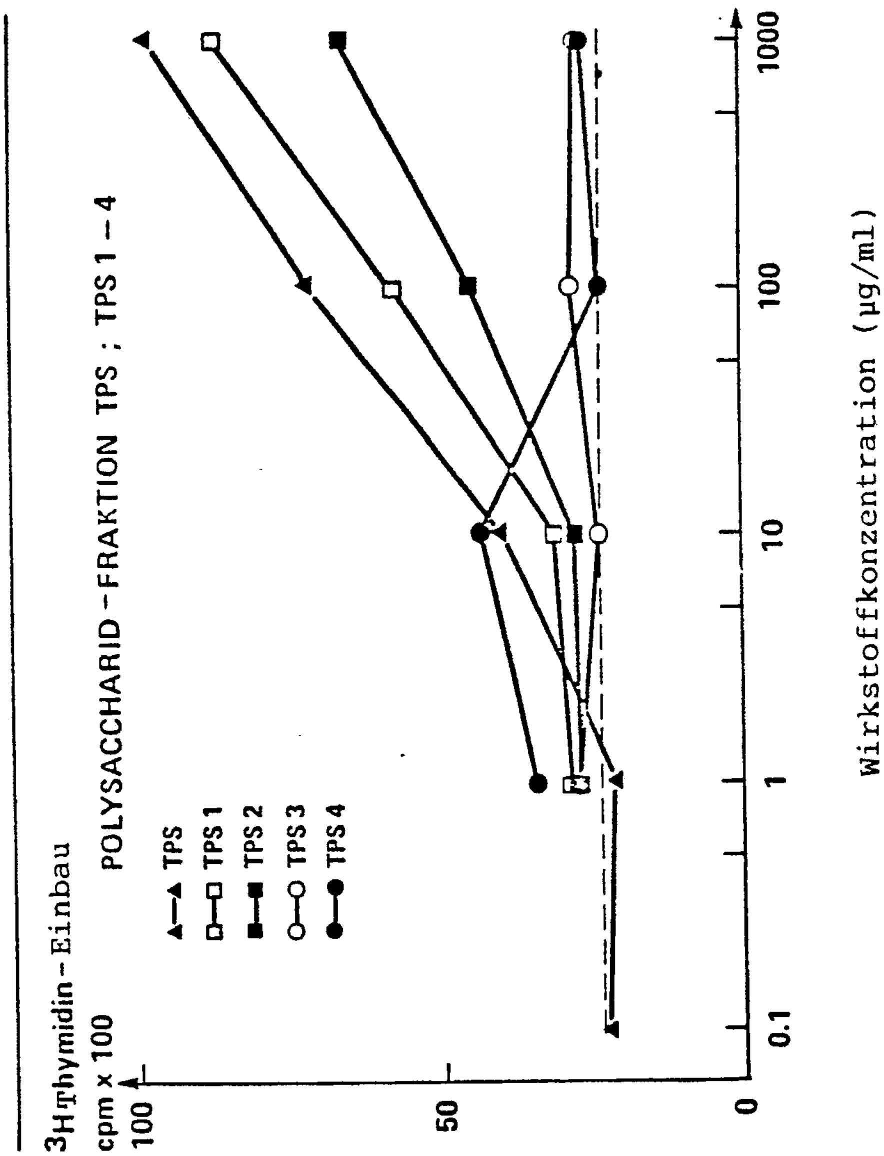

FIGUR 4

Mitogen-Nachweis (Kinetik)

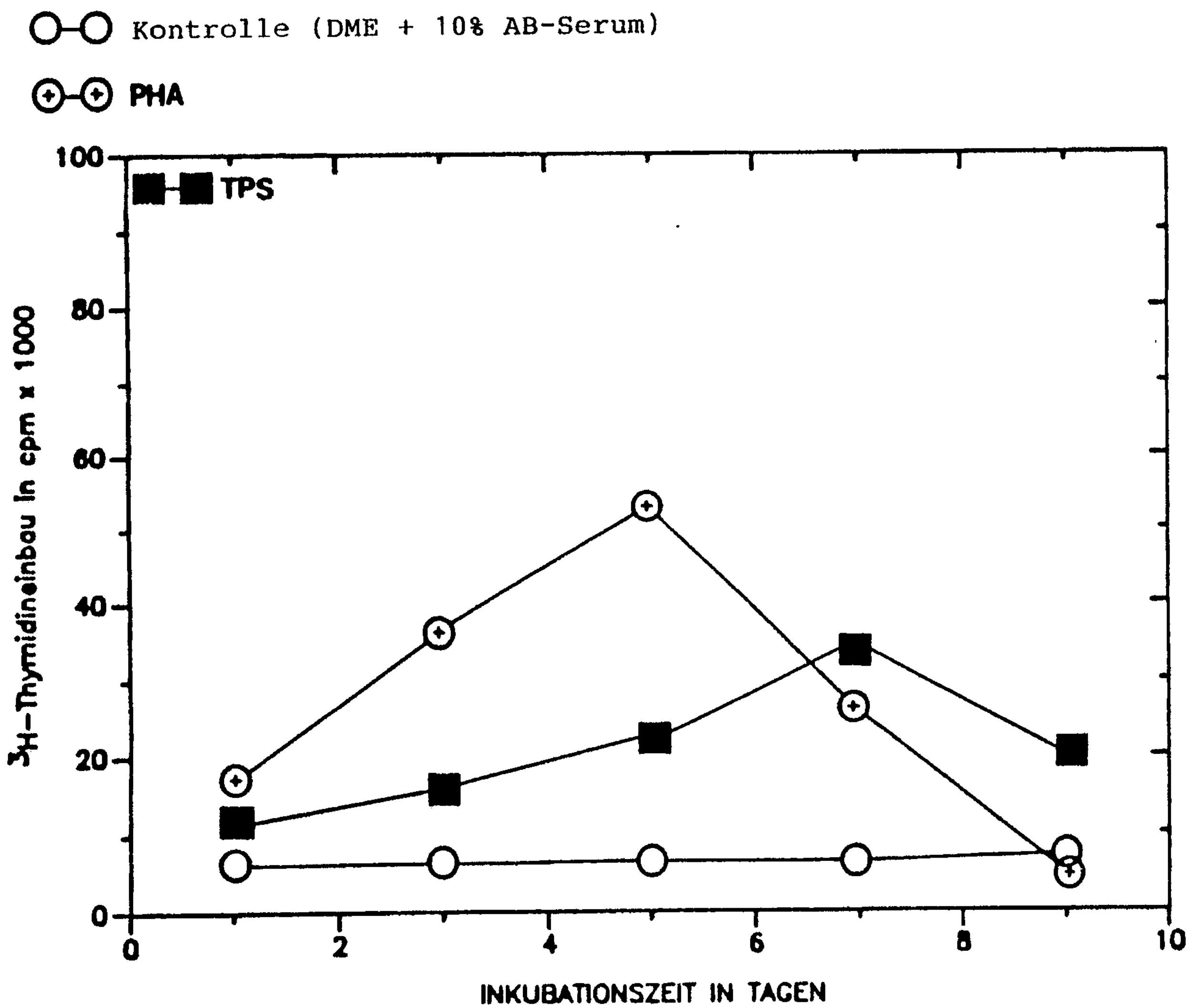

FIGUR 5

NK-Zell-Assay

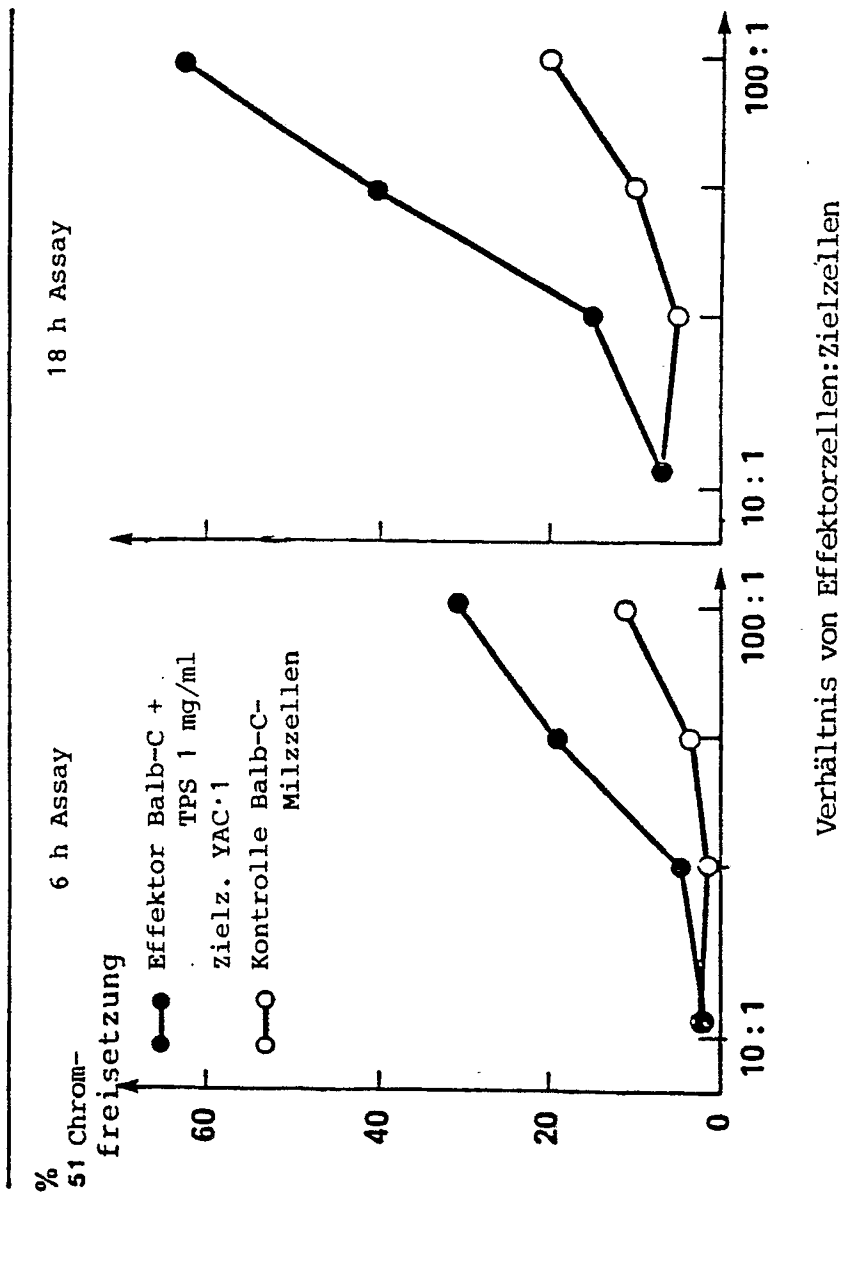

FIGUR 6

Biolumineszenz-Nachweis

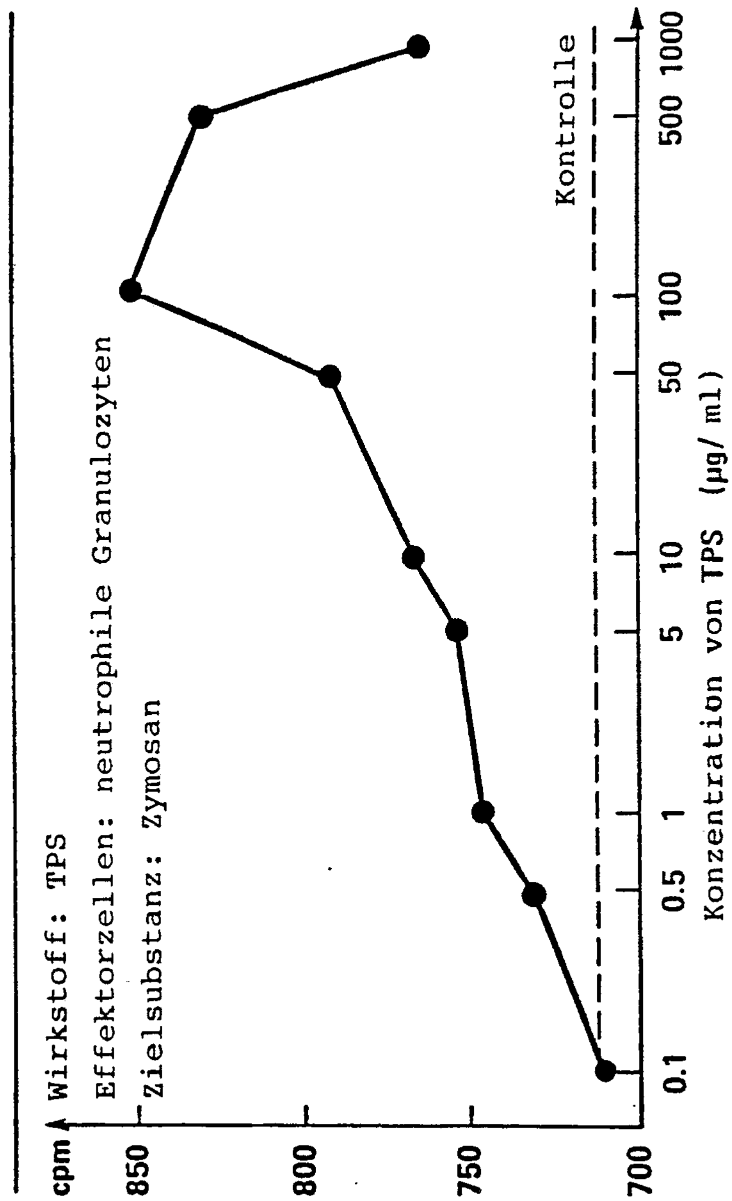

FIGUR 7

Phagozytoseassay neutrophiler Granulozyten

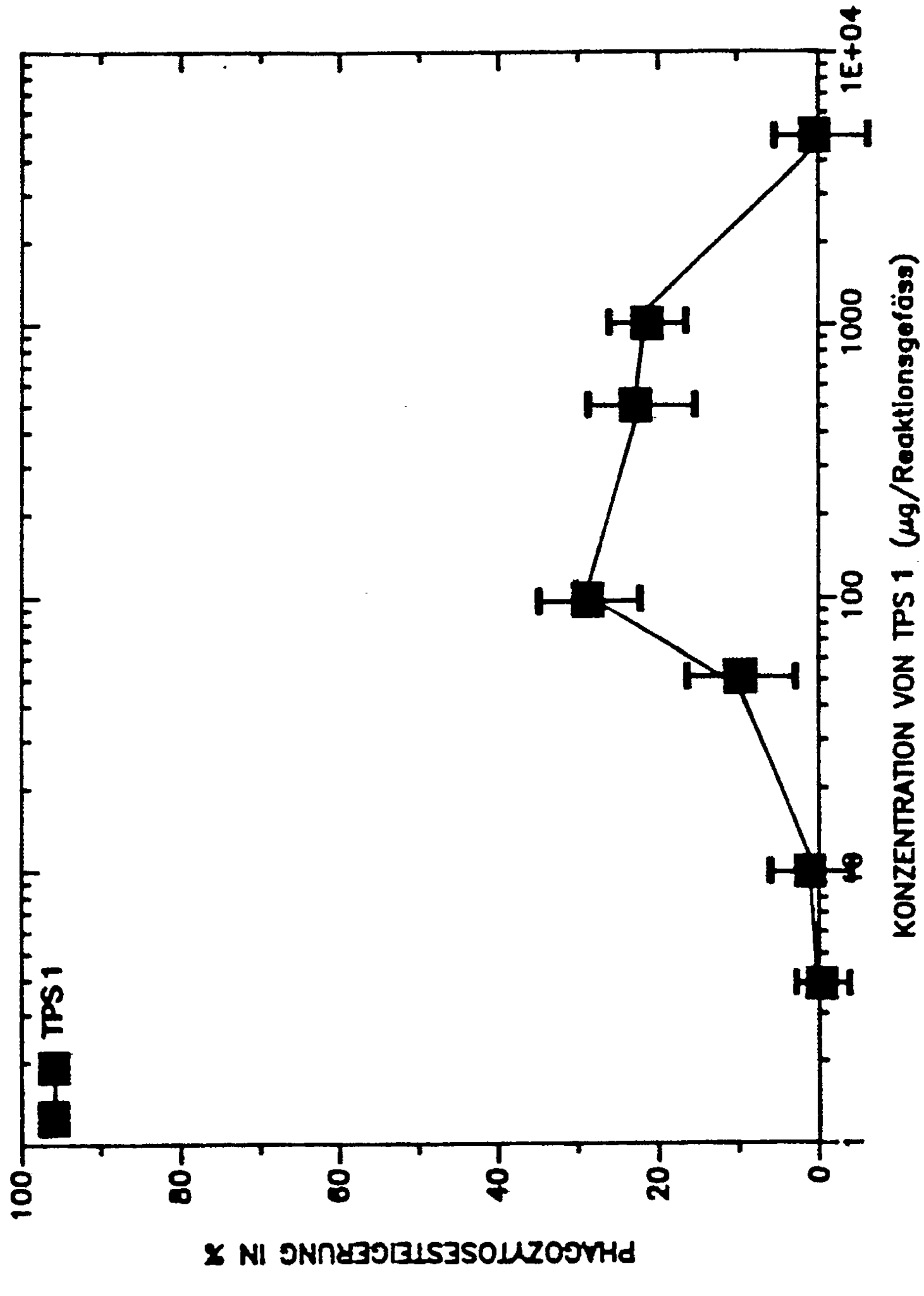

FIGUR 8

Phagozytoseassay neutrophiler Granulozyten

Zielzellen- Candida albicans

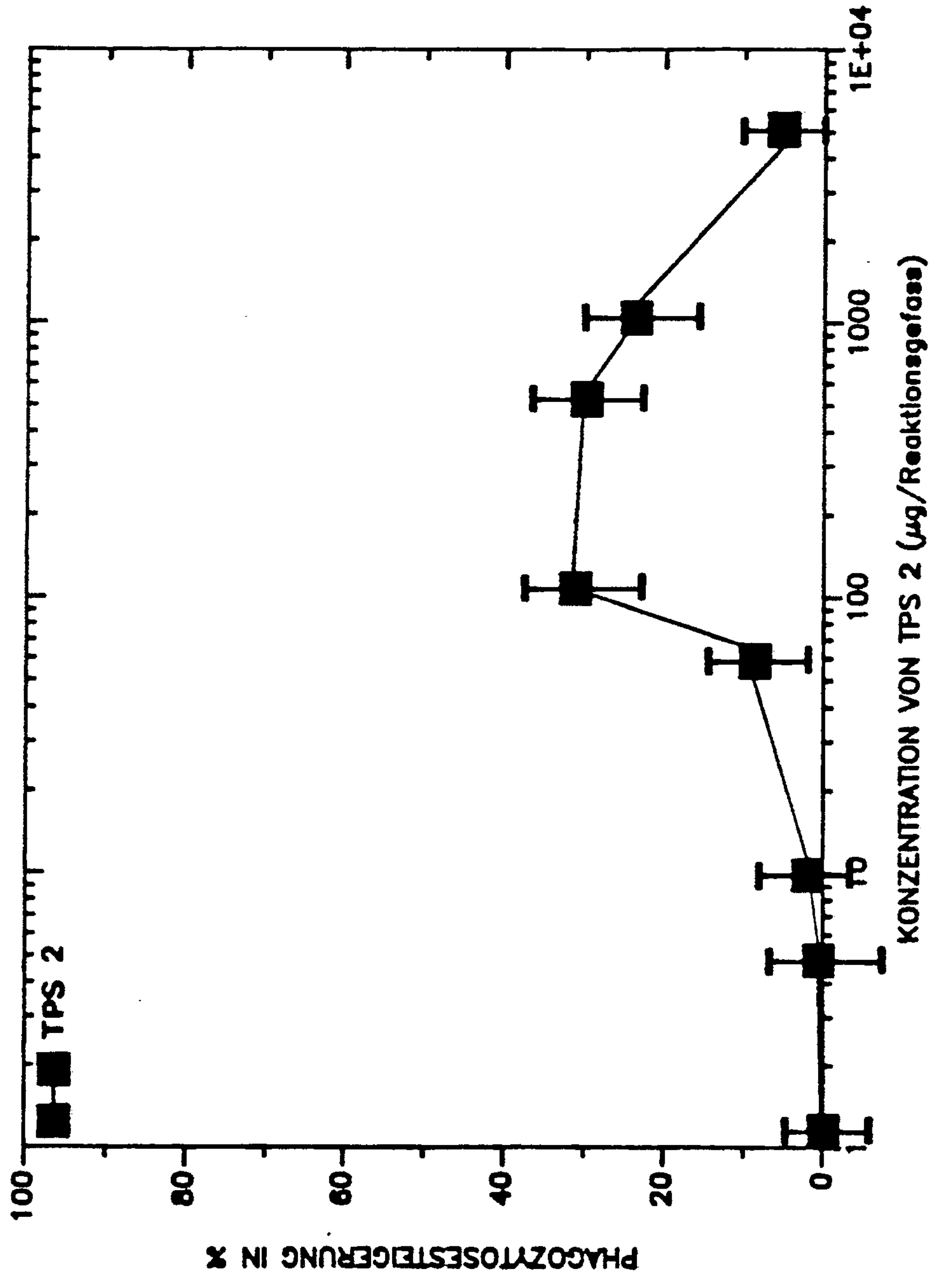

FIGUR 9

TPS wirkt nicht mitogen auf angereicherte B-Zellen

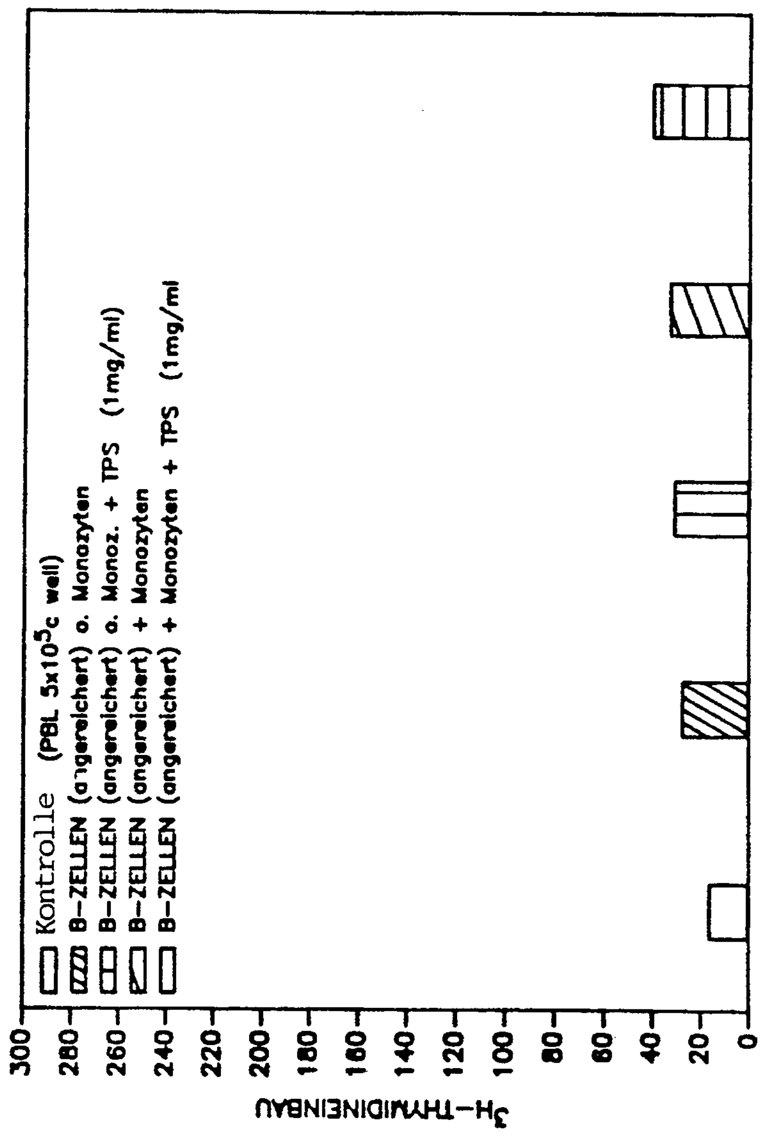

FIGUR 10

Mitogene Wirkung von TPS auf T-Zellen

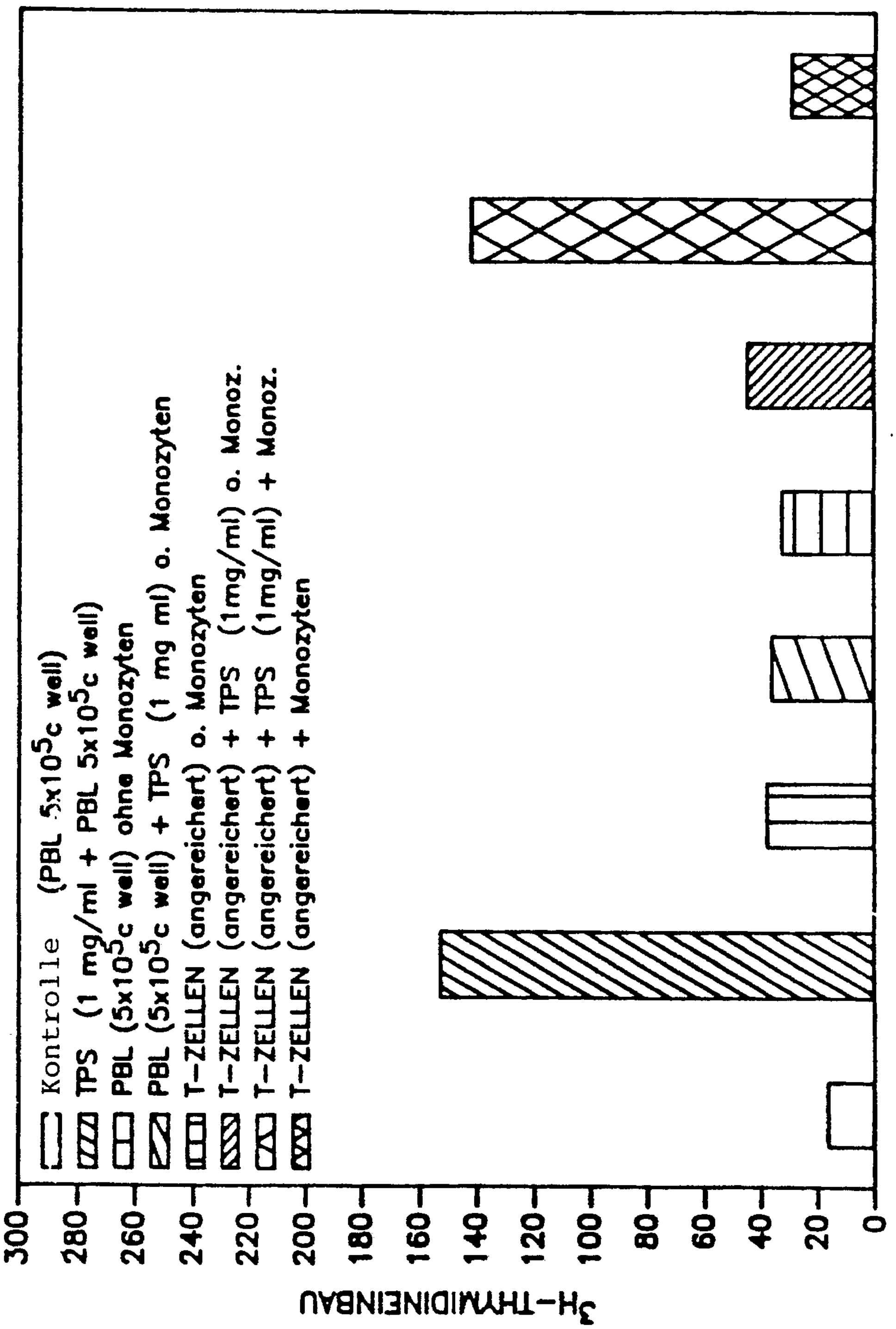

FIGUR 11

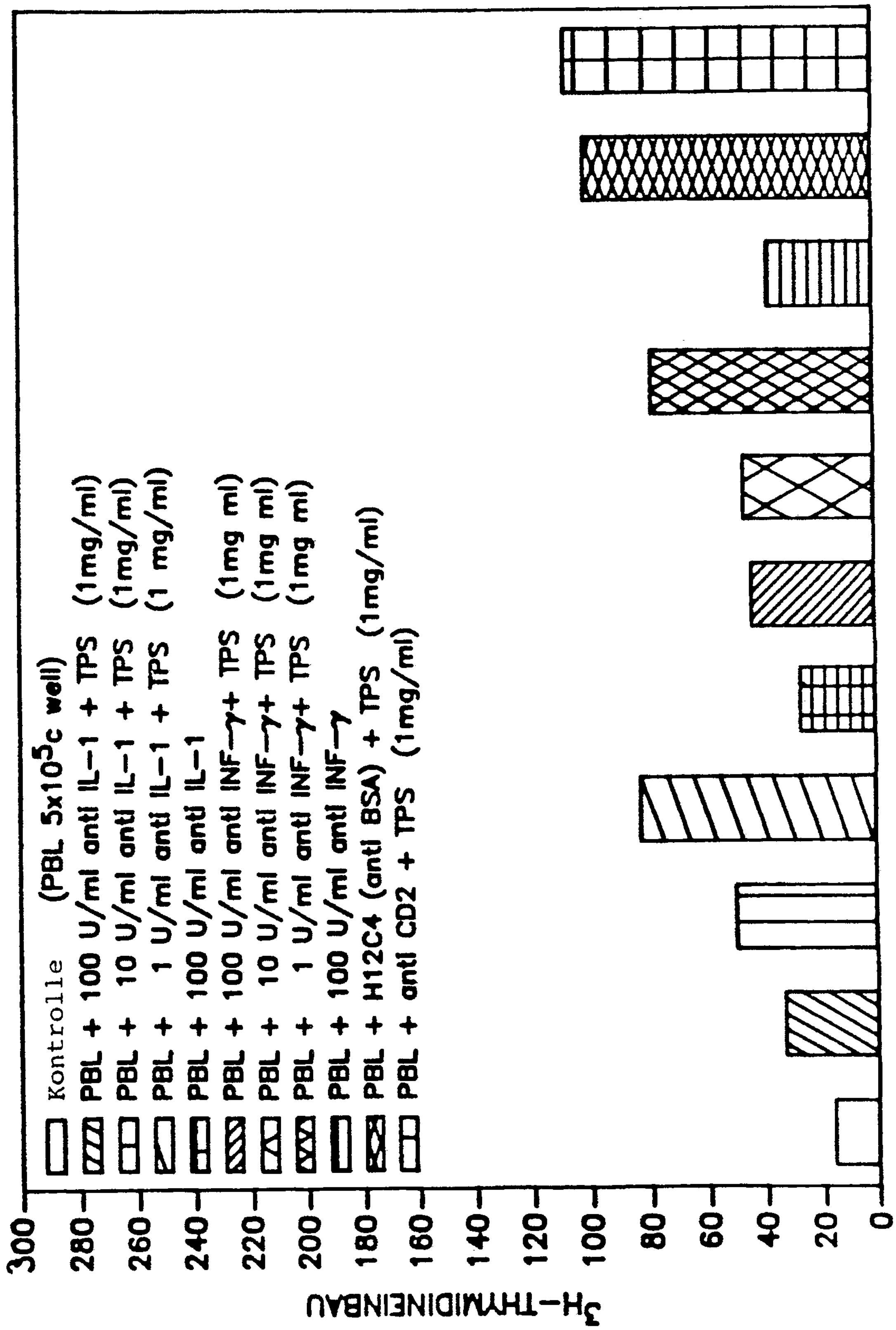
Kontrolle (PBL 5x10^5c well)
PBL + 100 U/ml anti IL-1 + TPS (1mg/ml)
PBL + 10 U/ml anti IL-1 + TPS (1mg/ml)
PBL + 1 U/ml anti IL-1 + TPS (1 mg/ml)
PBL + 100 U/ml anti IL-1
PBL + 100 U/ml anti INF-γ+ TPS (1mg ml)
PBL + 10 U/ml anti INF-γ+ TPS (1mg ml)
PBL + 1 U/ml anti INF-γ+ TPS (1mg ml)
PBL + 100 U/ml anti INF-γ
PBL + H12C4 (anti BSA) + TPS (1mg/ml)
PBL + anti CD2 + TPS (1mg/ml)
3H-THYMIDINEINBAU
300
280
260
240
220
200
180
160
140
120
100
80
60
40
20
0

FIGUR 12

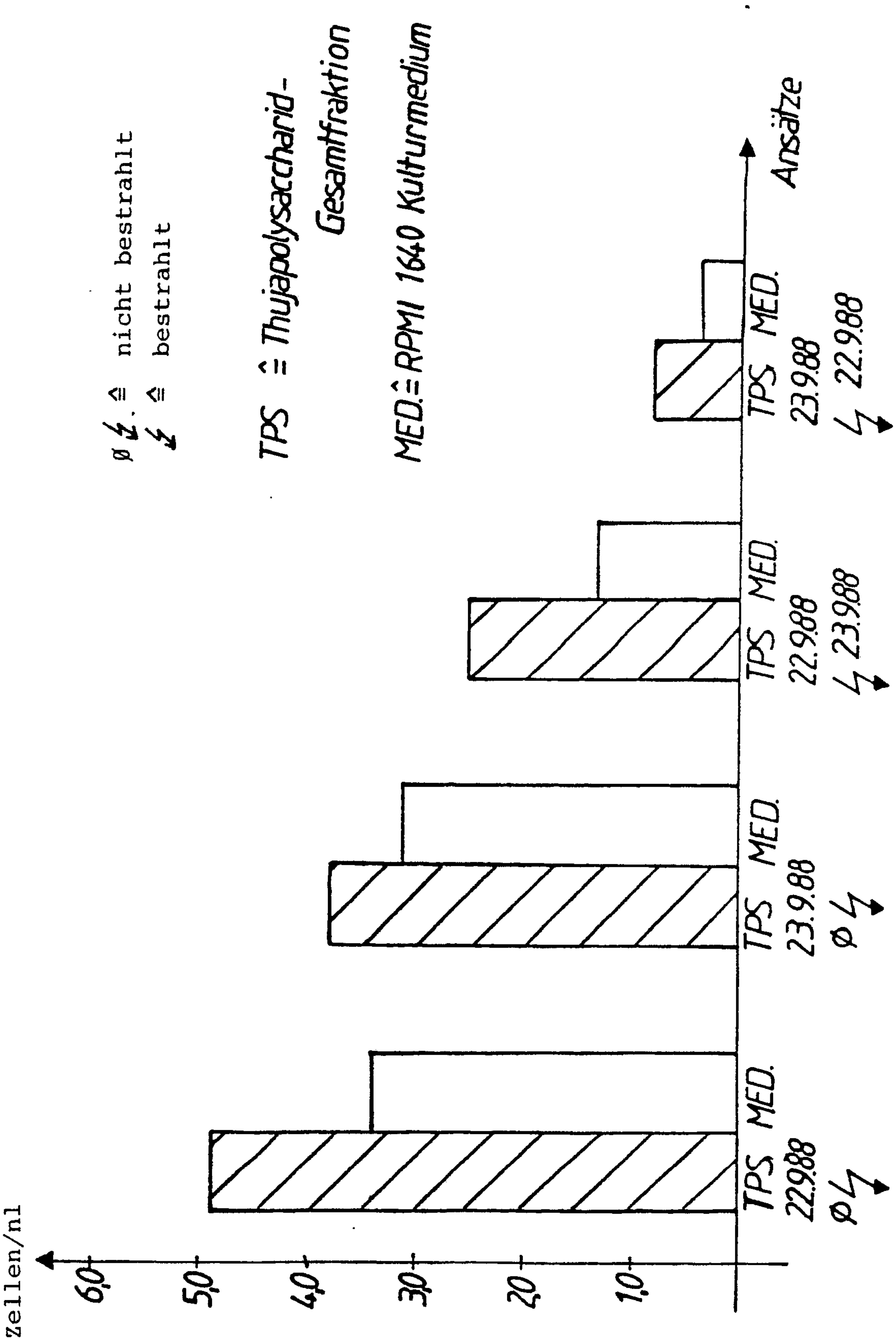
Zellen/nl
6,0
5,0
4,0
3,0
2,0
1,0
Ansätze
Ø ↯. ≙ nicht bestrahlt
↯ ≙ bestrahlt
TPS ≙ Thujapolysaccharid-Gesamtfraktion
MED. ≙ RPMI 1640 Kulturmedium
TPS MED.
22.9.88
Ø ↯
TPS MED.
23.9.88
Ø ↯
TPS MED.
22.9.88
↯ 23.9.88
TPS MED.
23.9.88
↯ 22.9.88

Abb. 1

Pappenheimfärbung pheripherer Blutleukozyten

Zellkultur: 4 Tage alt
Vergrößerung 630-fach

Unstimuliert

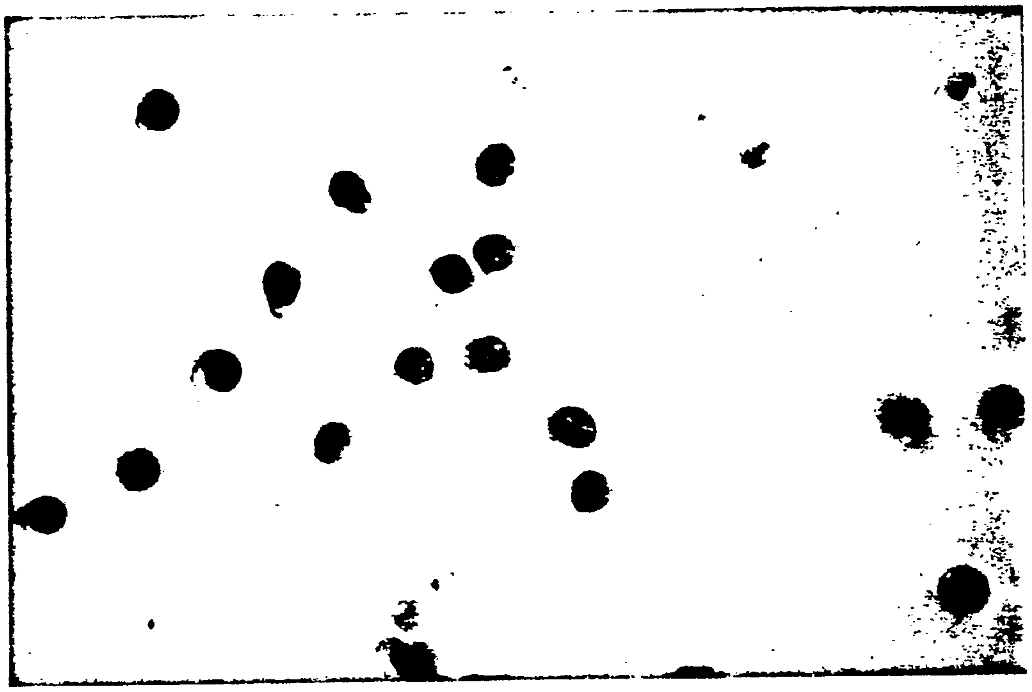

Stimuliert mit 1 mg/ml TPS

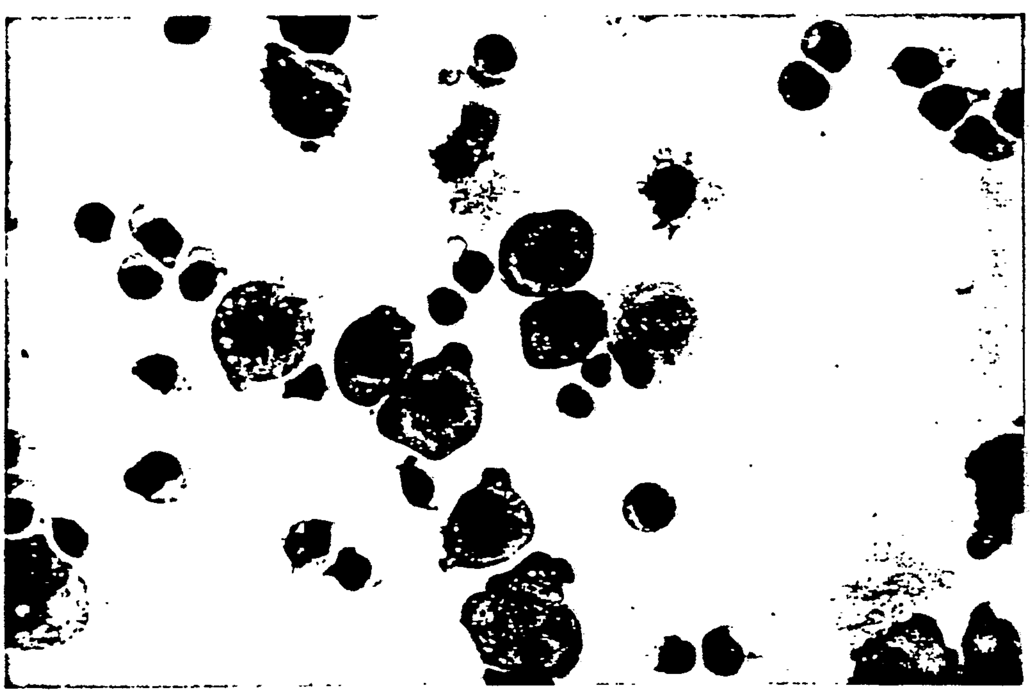

Abb. 2

APAAP-Färbung pheripherer Blutleukozyten

Zellkultur: 4 Tage alt
Vergrößerung 630-fach
Stimulation: TPS 1 mg/ml

Antikörper: anti-$CD_8$ (T-Zell negativ)

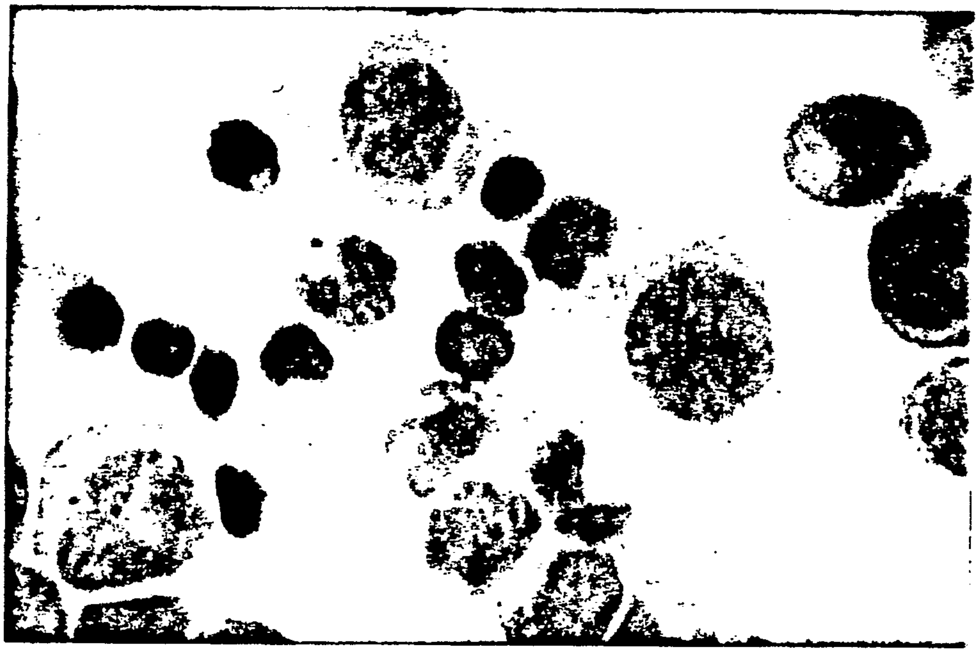

Antikörper: anti-$CD_4$ (T-Zell positiv)